# EUROPEAN PATENT SPECIFICATION

(11) **EP 2 569 445 B1**
(45) Date of publication and mention of the grant of the patent: **04.01.2017**
(21) Application number: 11780031.8
(22) Date of filing: 12.05.2011
(51) Int. Cl.: C12Q 1/34, C12Q 1/44, C12Q 1/68, G01N 33/50

(54) **SCREENING ASSAYS BASED ON ABHD6 FOR SELECTING INSULIN SECRETION PROMOTING AGENTS**
SCREENING-ASSAYS AUF BASIS VON ABHD6 ZUR AUSWAHL INSULINAUSSCHEIDUNGSFÖRDERNDER MITTEL
DOSAGES PAR CRIBLAGE BASÉS SUR ABHD6 POUR SÉLECTIONNER DES AGENTS FAVORISANT LA SÉCRÉTION D'INSULINE

(30) Priority: 12.05.2010 US 333772 P
(43) Date of publication of application: 20.03.2013
(73) Proprietor: Val-Chum, Limited Partnership, Montréal, QC H3V 1H8 (CA)
(72) Inventor: MADIRAJU, Murthy, Brossard Québec J4W 2W2 (CA); PRENTKI, Marc, Ville Mont-Royal Québec H3P 1R2 (CA); JOLY, Erik, Blainville Québec J7C 5R1 (CA)
(74) Representative: Cabinet Plasseraud
(86) International application number: PCT/CA2011/050295
(87) International publication number: WO 2011/140659

(56) References cited:
- Shangang Zhao: "Monoacylglycerol as a metabolic coupling factor in glucose-stimulated insulin secretion", Mémoire présentée à la Faculté des Etudes Supérieures en vue de l'obtention du grade de maître ès sciences en Biochimie, 1 December 2010 (2010-12-01), pages 1-133, XP055086701, Université de Montréal Retrieved from the Internet: URL:http://www.ncbi.nlm.nih.gov/pubmed/240 70767 [retrieved on 2013-11-05]
- BERMUDEZ-SILVA, F.J. ET AL.: 'Presence of functional cannabinoid receptors in human endocrine pancreas' DIABETOLOGIA vol. 51, 2008, pages 476 - 487, XP019582955
- BLANKMAN J. ET AL: 'A Comprehensive Profile of Brain Enzymes that Hedrolyze the Endocannabinoid 2-Arachidonoylglycerol' CHEMISTRY & BIOLOGY vol. 14, no. 12, December 2007, pages 1347 - 1356, XP022392658

## Description

### BACKGROUND

Incidence of type-2 diabetes (T2D) and associated health problems worldwide has reached astronomical proportions and it has been recognized that reducing the hyperglycemia in T2D patients decreases the morbidity substantially and improves the quality of life. The ever increasing epidemic of obesity is a primary risk factor for the development of T2D as obesity is tightly linked with gradual increase in insulin resistance followed by the failure of β-cells to compensate and secrete sufficient insulin to control glycemia. Therapeutic management of T2D currently is achieved by drugs that either reduce insulin resistance, reduce liver gluconeogenesis or elevate insulin secretion by β-cells in order to control blood glucose levels. Remarkable progress has been made in the last decade in deducing the mechanisms of fuel-stimulated insulin secretion (IS) in the pancreatic β-cell and while the role of enhanced Ca²⁺ influx in the triggering of K_{ATP}-dependent pathway of glucose stimulated insulin secretion (GSIS) is established, the signaling molecules implicated in the amplification of K_{ATP}-independent pathway(s) remain to be defined. Much support has been provided for the concept that lipid mediators and glycerolipid/free fatty acid (GL/FFA) cycling, which is glucose-responsive in the β-cell, play key role in GSIS. GL/FFA cycling refers to the cyclic process of FFA esterification with glycerol to synthesize GL, followed by its hydrolysis releasing the FFA that can be re-esterified.

GL/FFA cycling is active in many cells allowing for continuous production of neutral (mono-, di- & tri-acylglycerols (MAG; DAG; TG)) and complex lipids and phospholipids (PL). Various intermediates of GL/FFA cycling including FFA, fatty acyl-CoAs (FACoA), DAG, etc., likely regulate GSIS, though the mechanisms by which they influence this process remain uncertain. The significance of GL/FFA cycling for insulin secretion became evident from studies showing curtailed GSIS in rat islets upon lipolysis inhibition by the pan-lipase inhibitors 3,5-dimethylpyrazole and orlistat, and also by the deletion of hormone-sensitive lipase (HSL) and adipose triglyceride lipase (ATGL). In the normoglycemic insulin resistant Zucker fatty rat, enhanced glucose-responsive GL/FFA cycling has been proposed to contribute to the hyperinsulinemia associated with sustained β-cell compensation of this animal. GL/FFA cycling intermediate, DAG, is thought to activate Munc-13-1, a vesicle priming protein, and also C-kinase enzymes, which play an important role in the exocytosis of insulin granules in β-cell. GL/FFA cycling and lipolysis derived monoacylglycerols as regulators of insulin secretion.

It would be highly desirable to be provided with novel signaling molecules implicated in the amplification/K_{ATP}-independent pathway(s). This novel signaling molecule could be useful to design novel screening assay for agents useful in the stimulation of insulin secretion. The agents identified by this method could be used in the treatment of diabetes as well as related conditions.

### SUMMARY

One aim of the present application is to provide tools to assess the ability of an agent to increase insulin secretion in pancreatic β-cells. As shown herein, agents capable of augmenting monoacylglyceride level at the inner surface of the cytoplasmic membrane of a cell or a cell membrane derived therefrom and/or inhibiting the activity of the ABHD6 polypeptide are considered useful for increasing insulin secretion.

According a first aspect, there is provided a method of characterizing an agent's ability to increase insulin secretion in a subject. Broadly, the method comprising extracting a value of a parameter of the ABHD6-based reagent in the presence of the agent, wherein the value is obtained by combining the agent with a ABHD6-based reagent in a reaction vessel, comparing the value of the parameter of the ABHD6-based reagent to a control value; and characterizing the agent. If the agent decreases the value of the parameter of the ABHD6-based reagent, then it is considered that it possesses the ability to increase insulin secretion in the subject. If the agent does not modify or increases the value of the parameter of the ABHD6-based reagent, then it is considered that it lacks the ability to increase insulin secretion in the subject. In this method, the ABHD6-based reagent is a ABHD6 polypeptide and the parameter is the ABHD6 polypeptide lipase activity, quantity or stability; or the ABHD6-based reagent is a polynucleotide encoding the ABHD6 polypeptide and the parameter is the level of expression or stability of the polynucleotide. In an embodiment, the ABHD6-based reagent is a ABHD6 polypeptide, and the parameter of the ABHD6-based reagent is a ABHD6 lipase activity. In another embodiment, the ABHD6-based reagent is a polynucleotide encoding a ABHD6 polypeptide, and the parameter of the ABHD6-based reagent is a level of expression of the polynucleotide. In an embodiment, the method further comprises adding the agent to a cell having the ABHD6-based reagent. The method may further comprise administering the agent to a non-human animal, such as a rodent. In still another embodiment, the control value is at least one of: the parameter of the ABHD6-based reagent in the absence of the agent, the parameter of the ABHD6-based reagent in the presence of a control agent that fails to increase insulin secretion in the subject and a pre-determined value associated with a lack of increase of insulin secretion. In a further embodiment, the subject is suffering from at least one of the following condition: diabetes (such as type II diabetes) and metabolic syndrome X.

Also described is the use of a comparison between a value of a parameter of a ABHD6-based reagent and a control to characterize an agent's ability to increase insulin secretion in a subject. In an embodiment, the ABHD6-based reagent is a ABHD6 polypeptide. The parameter of the ABHD6-based reagent may be a ABHD6 lipase activity. The ABHD6-based reagent may be a polynucleotide encoding a ABHD6 polypeptide. The parameter of the ABHD6-based reagent may be a level of expression of the polynucleotide. In another embodiment, the value of the parameter of the ABHD6-based reagent may be determined in a cell having the ABHD6-based reagent and/or in a non-human animal (such as a rodent). The control value may be at least one of: the parameter of the ABHD6-based reagent in the absence of the agent, the parameter of the ABHD6-based reagent in the presence of a control agent that fails to increase insulin secretion in the subject and a pre-determined value associated with a lack of increase of insulin secretion. The subject may be suffering from at least one of the following condition: diabetes (such as type II diabetes) and metabolic syndrome X.

According to another aspect, there is provided with a screening system for characterizing an agent's ability to increase insulin secretion in a subject. Broadly, the system comprises a reaction vessel for combining the agent with a ABHD6-based reagent, a processor in a computer system, a memory accessible by the processor, wherein the processor is configured to perform a computer-based method to carry out the steps of receiving a value of a parameter of the ABHD6-based reagent extracted from the ABHD6-based reagent in the presence of the agent; comparing the value of the parameter of the ABHD6-based reagent to a control value; and characterizing the agent as having the ability to increase insulin secretion in the subject when the value of the parameter of the ABHD6-based reagent is lower than the control value and as lacking the ability to increase insulin secretion in the subject when the value of the parameter of the ABHD6-based reagent is equal to or higher than the control value. In this system, the ABHD6-based reagent is a ABHD6 polypeptide and the parameter is the ABHD6 polypeptide lipase activity, quantity or stability, or the ABHD6-based reagent is a polynucleotide encoding the ABHD6 polypeptide and the parameter is the level of expression or stability of the polynucleotide. In an embodiment, the ABHD6-based reagent is a ABHD6 polypeptide, and the parameter of the ABHD6-based reagent is a ABHD6 lipase activity. In another embodiment, the ABHD6-based reagent is a polynucleotide encoding a ABHD6 polypeptide, and the parameter of the ABHD6-based reagent is a level of expression of the polynucleotide. In another embodiment, the value of the parameter of the ABHD6-based reagent is determined in a cell having the ABHD6-based reagent and/or in a non-human animal (such as a rodent). In still another embodiment, the control value is at least one of: the parameter of the ABHD6-based reagent in the absence of the agent, the parameter of the ABHD6-based reagent in the presence of a control agent that fails to increase insulin secretion in the subject and a pre-determined value associated with a lack of increase of insulin secretion. In a further embodiment, the subject is suffering from at least one of the following condition: diabetes (such as type II diabetes) and metabolic syndrome X.

In another aspect, there is provided a software product embodied on a computer readable medium and comprising instructions for characterizing an agent's ability to increase insulin secretion in a subject. Broadly, the software product comprises a receiving module for receiving a value of a parameter of a ABHD6-based reagent in the presence of the agent in a reaction vessel; a comparison module for determining if the value of the parameter of the ABHD6-based reagent in the presence of the agent is lower than, equal to or higher than a control value and generating a corresponding output and a characterization module receiving the corresponding output from the comparison module and adapted to characterize the usefulness of the agent for increasing insulin secretion. In this characterization module, the agent is characterized as having the ability to increase insulin secretion in the subject when the value of the parameter of the ABHD6-based reagent is lower than the control value and the agent is characterized as lacking the ability to increase insulin secretion in the subject when the value of the parameter of the ABHD6-based reagent is equal to or higher than the control value. In the software product, the ABHD6-based reagent is a ABHD6 polypeptide and the parameter is the ABHD6 polypeptide lipase activity, quantity or stability, or the ABHD6-based reagent is a polynucleotide encoding the ABHD6 polypeptide and the parameter is the level of expression or stability of the polynucleotide. In an embodiment, the ABHD6-based reagent is a ABHD6 polypeptide, and the parameter of the ABHD6-based reagent is a ABHD6 lipase activity. In another embodiment, the ABHD6-based reagent is a polynucleotide encoding a ABHD6 polypeptide, and the parameter of the ABHD6-based reagent is a level of expression of the polynucleotide. In another embodiment, the value of the parameter of the ABHD6-based reagent is determined in a cell having the ABHD6-based reagent and/or in a non-human animal (such as a rodent). In still another embodiment, the control value is at least one of: the parameter of the ABHD6-based reagent in the absence of the agent, the parameter of the ABHD6-based reagent in the presence of a control agent that fails to increase insulin secretion in the subject and a pre-determined value associated with a lack of increase of insulin secretion. In a further embodiment, the subject is suffering from at least one of the following condition: diabetes (such as type II diabetes) and metabolic syndrome X.

Also described is a method of characterizing an agent's ability to increase insulin secretion in a subject. Broadly said method comprises combining the agent with a cell or a cell membrane, extracting a value of a level of monoacylglyceride (MAG) at the inner surface of the cytoplasmic membrane of the cell or the cell membrane in the presence of the agent, comparing the value of the level of MAG to a control value and characterizing the agent. When the agent increases the value of the level MAG with respect to the control value, it is considered that the agent is able to increase insulin secretion in the subject. When the agent does not modulate or decreases the value of the level MAG with respect to the control value, it is considered that the agent lacks the ability to increase insulin secretion in the subject. MAG may be 2-monoacylglycerol. The method may further comprise adding the agent to a cultured cell. The cell may be a pancreatic β-cell. The method may further comprise measuring the level of MAG in a culture medium of the cell. The control value may be at least one of: the level of MAG in the absence of the agent, the level of MAG in the presence of a control agent that fails to increase insulin secretion in the subject and a pre-determined value of a level of MAG associated with a lack of increase of insulin secretion. The subject may be suffering from at least one of the following condition: diabetes (such as type II diabetes) and metabolic syndrome X.

Also described is a screening system for characterizing an agent's ability to increase insulin secretion in a subject. Broadly, the screening system comprises a reaction vessel for combining the agent with a cell or a cell membrane; a processor in a computer system; a memory accessible by the processor; and at least one application coupled to the processor. The at least one application is configured for extracting a value of a level of monoacylglyceride (MAG) at the inner surface of the cytoplasmic membrane of the cell or the cell membrane in the presence of the agent; comparing the value of the level of MAG of to a control value; and characterizing the agent as having the ability to increase insulin secretion in the subject when the value of the level of MAG is higher than the control value and as lacking the ability to increase insulin secretion in the subject when the value of the level of MAG is equal to or lower than the control value. MAG may be 2-monoacylglycerol. The method may further comprise adding the agent to a cultured cell. The cell may be a pancreatic β-cell. The method may further comprise measuring the level of MAG in a culture medium of the cell. The control value may be at least one of: the level of MAG in the absence of the agent, the level of MAG in the presence of a control agent that fails to increase insulin secretion in the subject and a pre-determined value of a level of MAG associated with a lack of increase of insulin secretion. The subject may be suffering from at least one of the following condition: diabetes (such as type II diabetes) and metabolic syndrome X.

Also described is a software product embodied on a computer readable medium and comprising instructions for characterizing an agent's ability to increase insulin secretion in a subject. Broadly, this software product comprises a receiving module for receiving a value of a level of monoacylglyceride (MAG) at the inner surface of the plasma membrane of a cell or a cell membrane in the presence of the agent, a comparison module for determining if the value of the level of MAG in the presence of the agent is lower than, equal to or higher than a control value and generating a corresponding output and a characterization module receiving the corresponding output from the comparison module and adapted to characterize the usefulness of the agent for increasing insulin secretion. When the agent increases the value of MAG with respect to a control value, the agent is characterized as having the ability to increase insulin secretion in the subject. When the agent does not modulate or decreases the value of MAG with respect to a control value, the agent is characterized as lacking the ability to increase insulin secretion in the subject. MAG may be 2-monoacylglycerol. The cell may be a cultured cell and/or a pancreatic β-cell. The method may further comprise measuring the level of MAG in a culture medium of the cell. The control value may be at least one of: the level of MAG in the absence of the agent, the level of MAG in the presence of a control agent that fails to increase insulin secretion in the subject and a pre-determined value of a level of MAG associated with a lack of increase of insulin secretion. The subject may be suffering from at least one of the following condition: diabetes (such as type II diabetes) and metabolic syndrome X.

Also described is the use of a comparison between a value of a level of MAG at the inner surface of the cytoplasmic membrane of a cell or a cell membrane and a control value to characterize an agent's ability to increase insulin secretion in a subject. MAG may be 2-monoacylglycerol. The cell may be a cultured cell and/or a pancreatic β-cell. The value of the level of MAG may be measured a culture medium of the cell. The control value may be at least one of: the level of MAG in the absence of the agent, the level of MAG in the presence of a control agent that fails to increase insulin secretion in the subject and a pre-determined value of a level of MAG associated with a lack of increase of insulin secretion. The subject may be suffering from at least one of the following condition: diabetes (such as type II diabetes) and metabolic syndrome X.

### BRIEF DESCRIPTION OF THE DRAWINGS

Having thus generally described the nature of the invention, reference will now be made to the accompanying drawings, and in which:

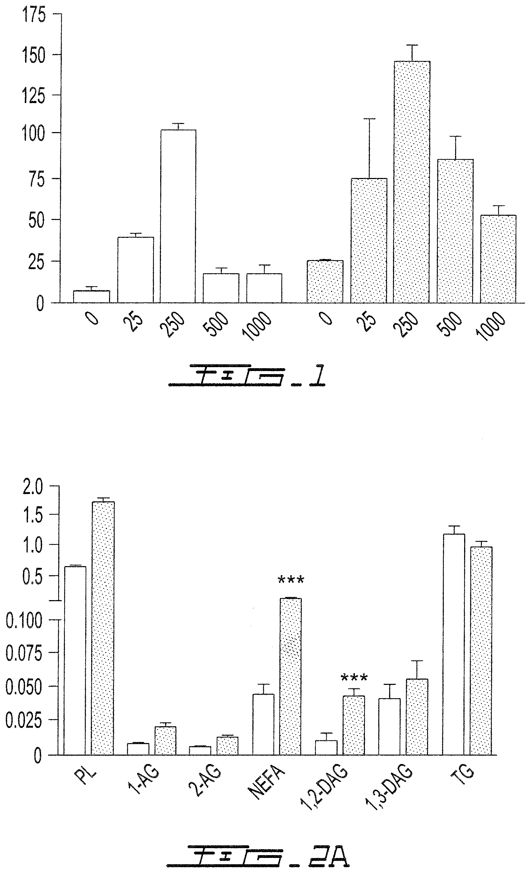

**Fig. 1** illustrates the concentration dependence of 2-AG stimulation of GSIS in INS 832/13 cells. Results are shown as ng insulin/mg protein/45 min in function of 2-AG concentration (nM shown on the X axis) and glucose concentration. 2-AG (2-arachidonylglycerol) at nanomolar concentration enhances insulin secretion at low (2 mM, 2G, white) and high (10 mM, 10G, dotted) glucose levels. However, the 2-AG effect declines with increase in its concentration (bell-shape curve). (n= 6-9; 3 separate expts).

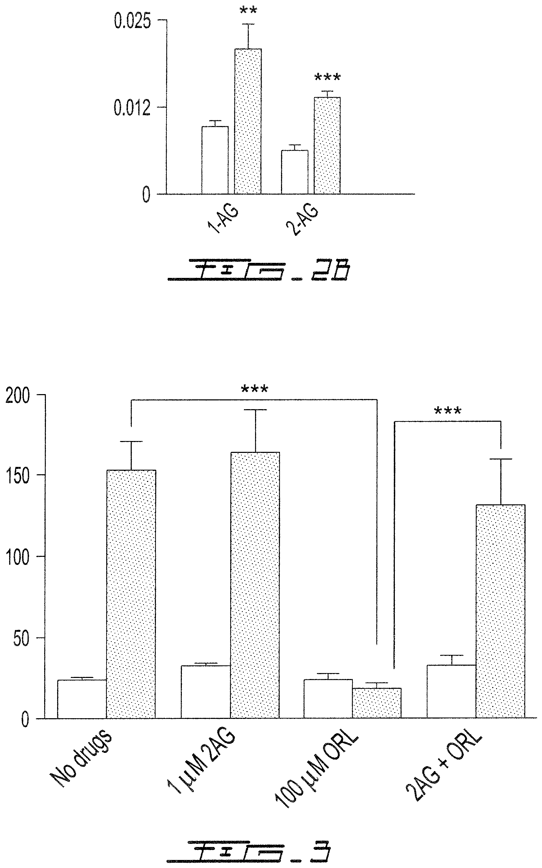

**Fig. 2** illustrates that glucose stimulates the incorporation of [1-¹⁴C]-arachidonic acid into 1-AG and 2-AG in INS 832/13 cells. Incorporation of [1-¹⁴C]-arachidonic acid (nmol/mg protein) is shown in function of various compounds. Results are shown for low (2 mM, 2G, white) and high (10 mM, 10G, dotted) glucose concentration. **(A)** Incubation of INS 832/13 cells with [1-¹⁴C]-arachidonic acid showed increased formation of 2-AG and 1-monoarachidonylglycerol (1-AG) at 10 mM glucose (10G), which is stimulatory for insulin secretion. (n=6; *** p<0.001). Results are shown in nmol/mg protein for different cellular compounds (PL = phospholipids, 1-AG = 1-monoarachidonylglycerol, 2-AG = 2-monoarachidonylglycerol, NEFA = non-esterified fatty acids, 1,2-DAG = 1,2-diacylglycerol, 1,3-DAG = 1,3-diacylglycerol, TG = triacylglycerol). **(B)** shows the same results that were presented in Figure 2A, but only for 1-AG and 2-AG, on a different scale.
**Fig. 3** illustrates that 2-AG restores GSIS inhibited by the pan-lipase inhibitor orlistat in INS 832/13 cells. Insulin secretion (ng insulin/mg protein/45 min) was measured in INS 832/13 cells incubated with a control (No drugs), 1 µM 2-AG (1 µM 2AG), 100 µm orlistat (100 µM ORL) or a combination of 1 µM 2-AG and 100 µM orlistat (2AG + ORL). The results presented in Figure 3 show that 2-AG is able to restore insulin secretion, that is inhibited by orlistat in INS 832/13 cells (2G = 2 mM glucose, white; 10G = 10 mM glucose, dotted; n=6-9; ***p<0.001).

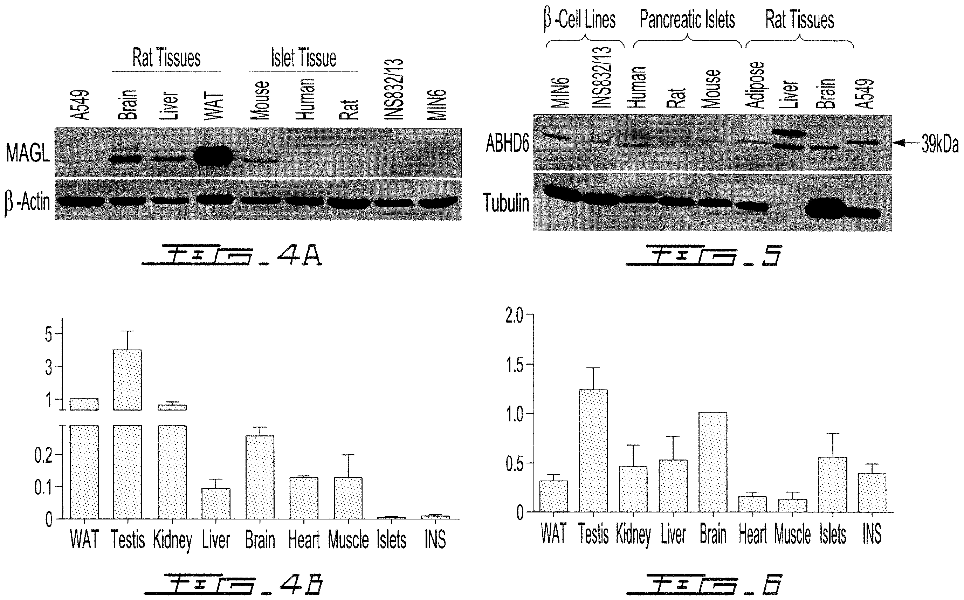

**Fig. 4** illustrates the expression profile **(A.** protein as assessed by Western Blot and **B.** mRNA as assessed by qRT-PCR) of MAG lipase (MAGL) in various A549 cells, rat tissues (brain, liver, WAT (white adipose tissue)), isolated islets (Islet tissue) for mouse human and rat, as well as INS 832/13 cells (INS) and MIN6 cells. In **(A),** results are shown in relative units of expression of MAG lipase/β-actin. In **(B),** results are shown in relative units of expression of MAG lipase/18S mRNA.
**Fig. 5** illustrates the expression profile (protein as assessed by Western Blot) of ABHD6 in various cells lines (MIN6 cells, INS832/13 cells, A549 cells), pancreatic islets from human, rat and mouse as well as rat tissues (white adipose tissue, liver, and brain). Results are shown in relative units of expression of ABHD6/tubulin. The arrow on the upper panel points to the 39kDa signal of the ABHD6 protein.
**Fig. 6** illustrates the expression profile (mRNA as assessed by qRT-PCR) of ABHD6 relative to 18S mRNA in various rat tissues (WAT = white adipose tissue, testis, kidney, liver, brain, heart, muscle, islets = pancreatic islets) and the INS832/13 cell line (INS).

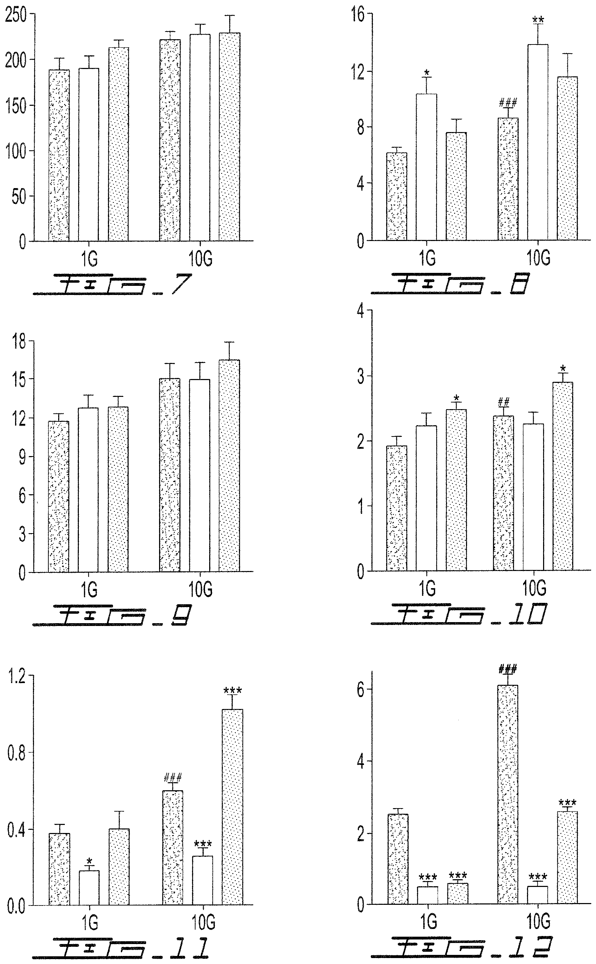

**Fig. 7** illustrates the effect of glucose and neutral glycerolipid lipase inhibitors on glucose incorporation into phospholipids in INS 832/13 cells. The cells were incubated at 1 (1 G) & 10 (10G) mM [U¹⁴C]-glucose in the presence of a control (DMSO, stippled), 50 µM orlistat (white) or 10 µM WWL70 (dotted). Results (shown in nmol glucose incorporated/mg of protein) indicate the amount of glucose used for phospholipid synthesis. None of the lipase inhibitors had any significant effect on phospholipids (n=9; from 3 separate expts).
**Fig. 8** illustrates the effect of glucose and neutral glycerolipid lipase inhibitors on glucose incorporation into triglycerides (TG) in INS 832/13 cells. The cells were incubated at 1 (1 G) & 10 (10G) mM [U¹⁴C]-glucose in the presence of a control (DMSO, stippled), 50 µM orlistat (white) or 10 µM WWL70 (dotted). Results (shown in nmol glucose incorporated/mg of protein) indicate the amount of glucose used for triglyceride synthesis. TG accumulated in the presence of orlistat while ABHD6 inhibition only had marginal effect on TG accumulation (n=9; from 3 separate expts; * p<0.05; ** p<0.01 as compared to corresponding DMSO control; ### p<0.001 compared to 1 G).
**Fig. 9** illustrates the effect of glucose and neutral glycerolipid lipase inhibitors on glucose incorporation into diacylglycerol (DAG) in INS 832/13 cells. The cells were incubated at 1 (1 G) & 10 (10G) mM [U¹⁴C]-glucose in the presence of a control (DMSO, stippled), 50 µM orlistat (white) or 10 µM WWL70 (dotted). Results (shown in nmol glucose incorporated/mg of protein) indicate the amount of glucose used for DAG synthesis. DAG levels include both 1,2- and 2,3-isomers. Neither of the inhibitors had any effect on DAG levels (n=9; from 3 separate expts).
**Fig. 10** illustrates the effect of glucose and neutral glycerolipid lipase inhibitors on glucose incorporation into 2-monoacylglycerol (2-MAG) in INS 832/13 cells. The cells were incubated at 1 (1 G) & 10 (10G) mM [U¹⁴C]-glucose in the presence of a control (DMSO, stippled), 50 µM orlistat (white) or 10 µM WWL70 (dotted). Results (shown in nmol glucose incorporated/mg of protein) indicate the amount of glucose used for 2-MAG synthesis. While orlistat showed no effect on 2-MAG, inhibition of either MAG-lipase or ABHD6 caused an elevation of this lipid (* p<0.05 compared to corresponding DMSO control; ## p<0.01 compared to 1 G (n=9; from 3 separate expts)).
**Fig. 11** illustrates the effect of glucose and neutral glycerolipid lipase inhibitors on glucose incorporation into 1-monoacylglycerol (1-MAG) in INS 832/13 cells. The cells were incubated at 1 (1 G) & 10 (10G) mM [U¹⁴C]-glucose in the presence of a control (DMSO, stippled), 50 µM orlistat (white) or 10 µM WWL70 (dotted). Results (shown in nmol glucose incorporated/mg of protein) indicate the amount of glucose used for 1-MAG synthesis. 1-MAG is formed mostly by the hydrolysis of LPA by lipid-P phosphatase and also DAG to a small extent. While orlistat significantly lowered 1-MAG (unlike 2-MAG), inhibition of either MAG lipase or ABHD6 caused an elevation of this lipid (* p<0.05; *** p<0.001 compared to corresponding DMSO control; ### p<0.001 compared to 1 G (n=9; from 3 separate expts)).
**Fig. 12** illustrates the effect of glucose and neutral glycerolipid lipase inhibitors on glucose incorporation into the release of [U¹⁴C]-free fatty acids (FFA) in INS 832/13 cells. The cells were incubated at 1 (1 G) & 10 (10G) mM [U¹⁴C]-glucose in the presence of a control (DMSO, stippled), 50 µM orlistat (white) or 10 µM WWL70 (dotted). Results (shown in FFA release/nmol glucose incorporated/mg of protein/2h) indicate the amount of FFA released. A significant portion of FFA arising mostly from lipid hydrolysis, which increases at high glucose, are released into extra-cellular medium. Orlistat almost completely lowered this release. However, inhibition of MAG lipase had no effect, even though this elevates MAG levels. Inhibition of ABHD6 on the other hand, decreases FFA release by about 2/3. This suggests that in INS cells, MAGL is probably present in the cytosol (not close to cell membrane) whereas ABHD6 is attached to membrane and generates FFA which are transported out rapidly. Therefore, the accumulation of MAG is in the interior of cytoplasm when MAGL is inhibited, while it is near cell membrane (and available for signaling) when ABHD6 is inhibited (*** p<0.001 compared to corresponding DMSO control; ### p<0.001 compared to 1G (n=9; from 3 separate expts)).

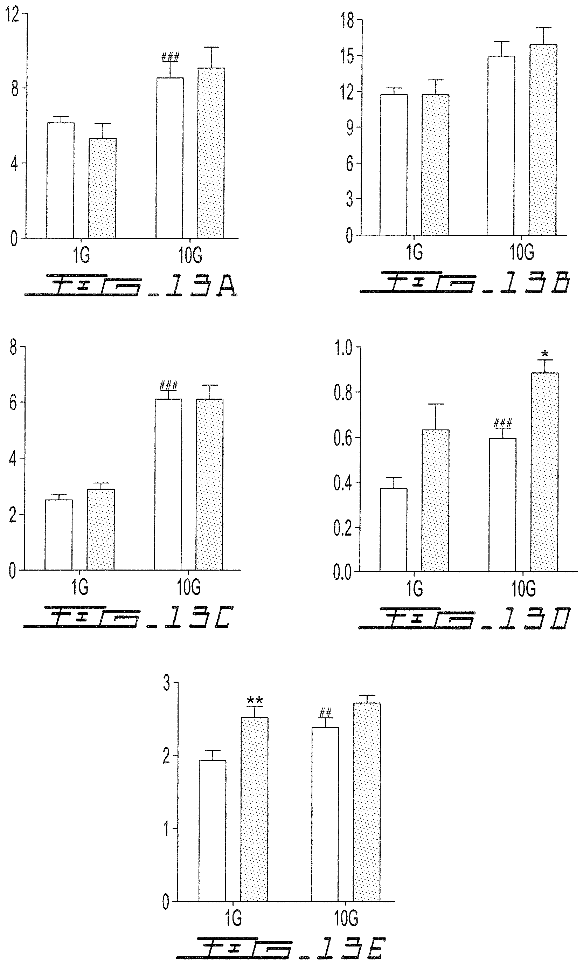

**Fig. 13** illustrates the effect of the MAG-lipase inhibitor JZL184 on [U¹⁴C]-glucose incorporation into various lipids in INS 832/13 cells. The cells were incubated at 1 (1 G) & 10 (10G) mM [U¹⁴C]-glucose in the presence of a control (DMSO, white) or 1 µM of JZL184 (dotted) **(A)** Results (shown in nmol glucose incorporated/mg of protein) indicate the amount of glucose used for triglyceride synthesis. **(B)** Results (shown in nmol glucose incorporated/mg of protein) indicate the amount of glucose used for DAG synthesis. DAG levels include both 1,2- and 2,3-isomers. **(C)** Results (shown in FFA release/nmol glucose incorporated/mg of protein/2h) indicate the amount of FFA released. **(D)** Results (shown in nmol glucose incorporated/mg of protein) indicate the amount of glucose used for 1-MAG synthesis. **(E)** Results (shown in nmol glucose incorporated/mg of protein) indicate the amount of glucose used for 2-MAG synthesis. MAG lipase inhibitor, JZL184 had no effect on glucose incorporation into TG, DAG and also the released FFA from the cells. However, both 1- & 2-MAG are elevated due to MAGL inhibition (* p<0.05; ** p<0.01 compared to corresponding DMSO control; ## p<0.01; ### p<0.001 compared to 1 G (n=9; from 3 separate expts)).

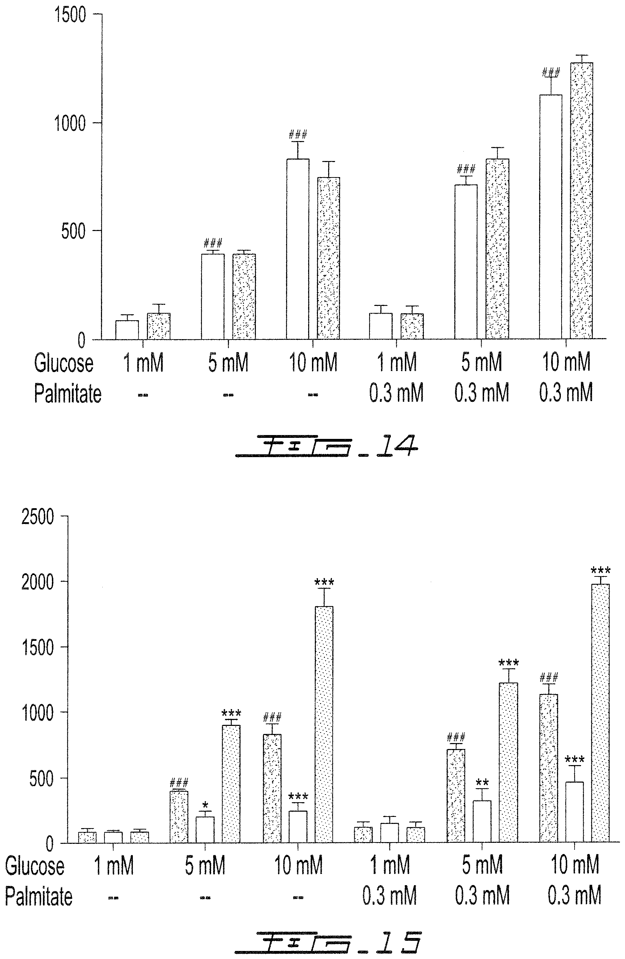

**Fig. 14** illustrates that the MAG lipase inhibitor, JZL184, has no effect on GSIS in INS 832/13 cells. Cells were incubated with a control (DMSO, white) or 1 µM JZL184 (MAGL inhibitor, stippled) in the presence of glucose (mM) or a combination of glucose and palmitate (mM). Concentration of glucose and palmintate are provided on the X axis of the graph. Insulin secretion was measured and is shown in ng/mgprotein/2h. As shown on this figure, there is a robust increase in GSIS with increasing glucose concentration but MAGL inhibitor, JZL184 had no effect (### p<0.001 compared to 1 mM Glucose (n=9; from 3 separate expts)).
**Fig. 15** illustrates that the ABHD6 inhibitor, WWL70, enhances GSIS in INS 832/13 cells. Cells were incubated a control (DMSO, stippled), 50 µM orlistat (white) or 10 µM WWL70 (doted) in the presence of glucose or a combination of glucose and palmitate. Concentration of glucose and palmintate are provided on the X axis of the graph. Insulin secretion was measured and is shown in ng/mgprotein/2h. As shown on this figure, there is a robust increase in glucose-stimulated insulin secretion by ABHD6 inhibitor, WWL70. This effect is specific to glucose and not to palmitate stimulation (* p<0.05; ** p<0.01; *** p<0.001 compared to corresponding DMSO control; ### p<0.001 compared to 1 mM glucose (n=9; from 3 separate expts)).

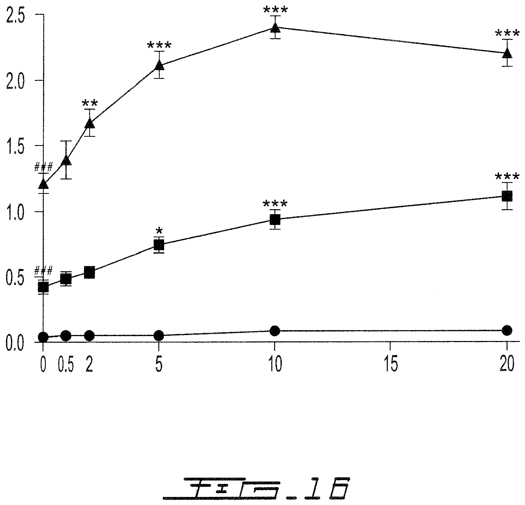

**Fig. 16** illustrates that the ABHD6 inhibitor, WWL70, enhances GSIS in INS 832/13 cells in a dose dependent manner. Cells were incubated at different glucose and WWL70 concentrations. Insulin secretion was assessed as mg/mgprotein/2h) in function of WWL70 concentration (in µM). Results are show at a 10mM concentration of glucose (▲), a 5mM concentration of glucose (■) or a 1 mM concentration of glucose (●). WWL70 shows optimal GSIS enhancing effect at about 10 mM concentration (* p<0.05; ** p<0.01; *** p<0.001 compared to corresponding vehicle control (no WWL70); ### p<0.001 compared to 1 mM Glucose (n=9; from 3 separate expts)).

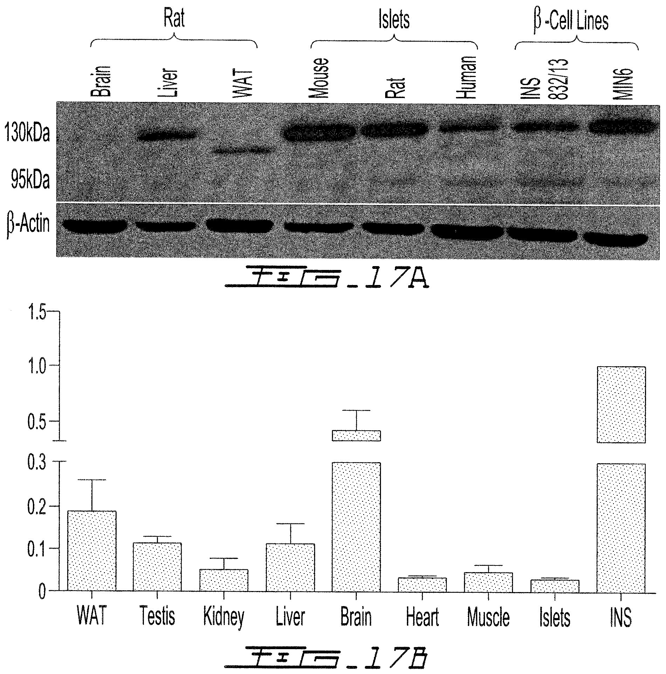

**Fig. 17** illustrates **(A)** the protein expression as assessed by Western Blot of glycosylated TRPV1-Receptor glycosylated (apparent weight 130kDa) or unglycosylated TRPV1-Receptor (apparent weight 95 kDa) in different rat tissues (Brain, Liver, WAT = white adipose tissue), isolated pancreatic islets (Mouse, Rat, Human) and cells (INS 832/13, MIN6) and **(B)** the expression of TRPV1-Receptor mRNA in different rat tissues (WAT = white adipose tissue, Testis, Kidney, Liver, Brain, Heart, Muscle, Islets = pancreatic islets) as will as in INS 832/13 cells (INS). Results are shown in relative units of expression of TRPV1-receptor/18S mRNA as assessed by qRT-PCR.

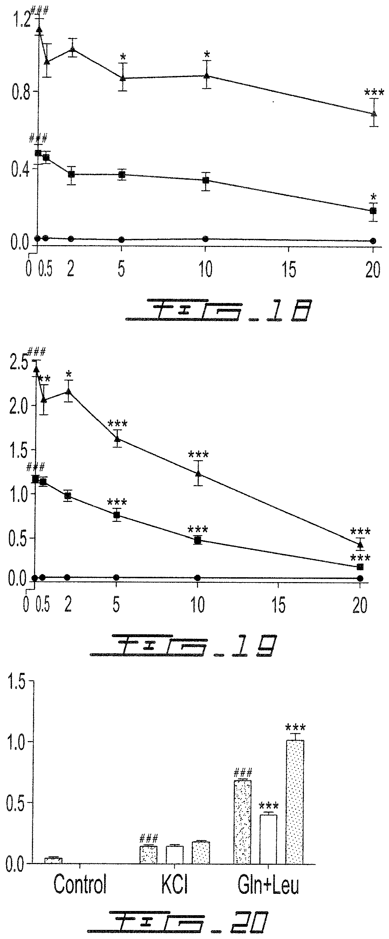

**Fig. 18** illustrates that a TRPV1-Receptor antagonist, AMG9810, dose-dependently reduces GSIS in INS 832/13 cells. Cells were incubated at different glucose (10 mM (▲), 5 mM (■), 1 mM (●)). Insulin secretion were measured and are shown in µg/mg protein/2h in function of AMG9810 concentrations (µM). A highly specific TRPV1 R-antagonist, AMG9810, antagonized the GSIS. This strongly implicates TRPV1 receptor in the regulation of insulin secretion (* p<0.05; *** p<0.001 compared to corresponding vehicle control (no AMG); ### p<0.001 compared to 1 mM Glucose (n=9; from 3 separate expts)).
**Fig. 19** illustrates that a TRPV1-R antagonist, AMG9810, dose-dependently inhibits WWL70-enhanced GSIS in INS 832/13 cells. Cells were incubated at different glucose (10 mM (▲), 5 mM (■) and 1 mM(●)) and WWL70 (10 µM). Insulin secretion were measured shown in µg/mg protein/2h in function of AMG9810 concentration (µM). TRPV1-R antagonist, AMG9810, completely antagonized the WWL70 stimulation at 10 µM. On its own, AMG9810 inhibits GSIS only at very high concentration (see Fig 18). This strongly implicates TRPV1 receptor in WWL70 effect on insulin secretion (* p<0.05; ** p<0.01; *** p<0.001 compared to corresponding vehicle control (no AMG); ### p<0.001 compared to 1 mM Glucose (n=9; from 3 separate expts)).
**Fig. 20** illustrates the effect of WWL70 and AMG9810 on insulin secretion stimulated by KCI and a combination of glutamine and leucine. Cells were incubated in the presence of a control (DMSO, stippled), 10 µM AMG9810 (white) or 10 µM WWL70 (dotted). Insulin secretion was measured and is shown as µg/mg protein/2h in function of control, incubation with KCI or a incubation with a combination of glutamine and leucine (Gln+Leu). The stimulatory effect of WWL70 was not seen when insulin secretion was induced by KCI (35 mM; glucose at 1 mM), which causes calcium influx (independent of TRPV1 R) and exocytosis. However, insulin secretion stimulated by amino acids, leucine + glutamine (5 mM, each) was enhanced by WWL70 and inhibited by AMG9810, suggesting that similar to GSIS, Gln+Leu stimulated insulin secretion is mediated in part via TRPV1 R-conducted Ca²⁺ influx Control, 1 mM glucose; n=9; *** p<0.001 compared to no drug (control) samples, ### p<001 compared to Control).

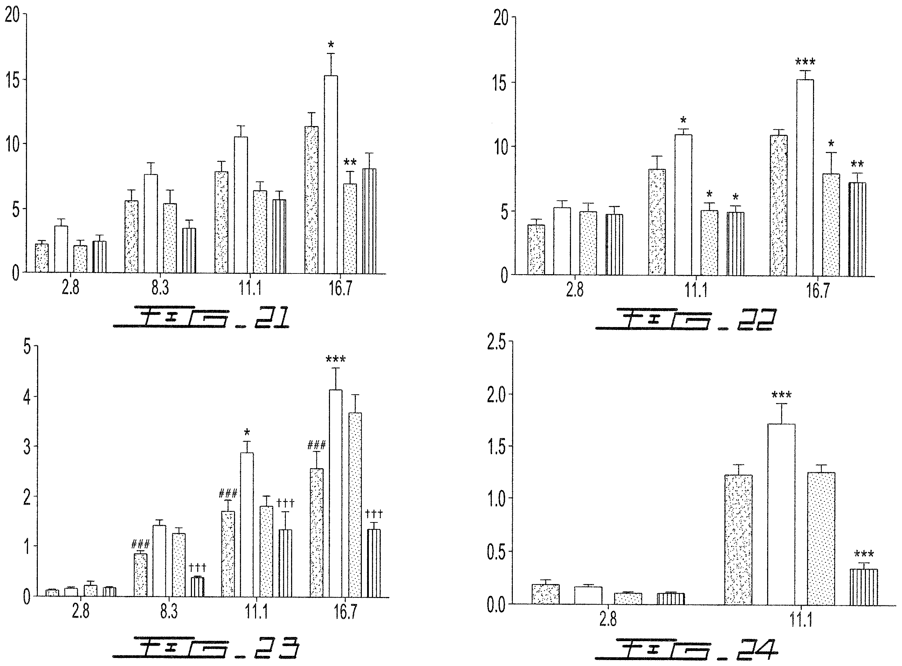

**Fig. 21** illustrates the effect of WWL70 and AMG9810 on GSIS in rat intact islets. The islets were incubated at different glucose concentrations (X axis, in mM) in the presence of a control (DMSO, stippled), 20 µM WWL70 (white), 20 µM AMG9810 (dotted) or a combination of 20 µM WWL70 and 20 µM AMG9810 (vertical lines). Insulin secretion was measured and is shown as % of total content in function of glucose concentration. In isolated intact rat islets WWL70 also enhanced GSIS and this is antagonized by AMG9810 (* p<0.05; ** p<0.01 compared to corresponding DMSO control (no drug) (n=12; from 3 separate expts)).
**Fig. 22** illustrates the effect of WWL70 and AMG9810 on GSIS in dispersed rat islet cells. The cells were incubated at different glucose concentrations (X axis, in mM) in the presence of a control (DMSO, stippled), 20 µM WWL70 (white), 20 µM AMG9810 (dotted) or a combination of 20 µM WWL70 and 20 µM AMG9810 (vertical lines). Insulin secretion was measured and is shown as % of total content/2h. WWL70 enhancement and AMG9810 inhibition of GSIS seen in intact isolated rat islets is also evident in dispersed rat islet cells, cultured for 24h. (n=9; 3 separate expts)). (* p<0.05; ** p<0.01, *** p<0.001 compared to corresponding DMSO control (no drug) (n=12; from 3 separate expts))
**Fig. 23** illustrates the effect of WWL70 and AMG9810 on GSIS in isolated CD1 mouse islets. The islets were incubated at different glucose concentrations (X axis, in mM) in the presence of a control (DMSO, stippled), 20 µM WWL70 (white), 20 µM AMG9810 (dotted) or a combination of 20 µM WWL70 and 20 µM AMG9810 (vertical lines). Insulin secretion was measured and is shown as % of total content/2h. Similar to rat islets, WWL70 enhanced GSIS in isolated intact islets from CD1-mouse and this is antagonized by AMG9810. This suggests that the mechanism is present in the β-cells from other species also. So, it is likely to operative *in vivo* (* p<0.05 compared to corresponding DMSO control; ** p<0.01 compared to corresponding DMSO control (no drug); ***p<0.001 compared to corresponding DMSO control, ### p<0.001 compared to 2.8 mM Glucose; ††† p<0.001 compared to WWL70 (n=9; from 3 separate expts)).
**Fig. 24** illustrates the effect of WWL70 and AMG9810 on GSIS in isolated C57BI6 mouse islets. The islets were incubated at different glucose concentrations (X axis, in mM) in the presence of a control (DMSO, stippled), 20 µM WWL70 (white), 20 µM AMG9810 (doted) or a combination of 20 µM WWL70 and 20 µM AMG9810 (vertical lines). Insulin secretion was measured and is shown as % of total content/2h. WWL70 enhancement and AMG9810 inhibition of GSIS were also seen in isolated intact islets from C57BI6 mouse (2.8G = 2.8 mM glucose, 11.1 G = 11.1 mM glucose, n=8; ***p<0.001 compared to no drug control).

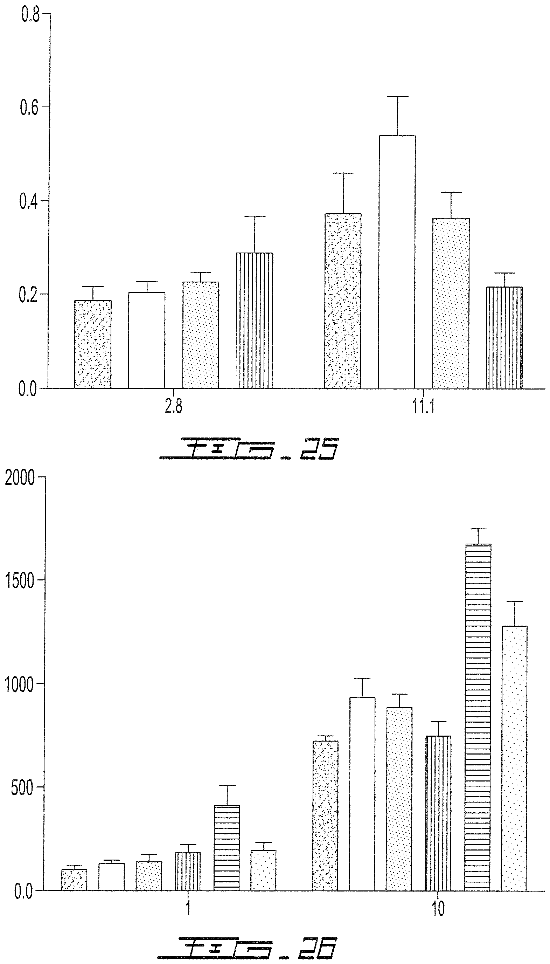

Fig. 25 illustrates the effect of WWL70 and AMG9810 on GSIS in human intact islets. The islets were incubated at different glucose concentrations (X axis, in mM) in the presence of a control (DMSO, stippled), 20 µM WWL70 (white), 20 µM AMG9810 (dotted) or a combination of 20 µM WWL70 and 20 µM AMG9810 (vertical lines). Insulin secretion was measured and is shown as % of total content/2h. The WWL70 enhancement and AMG9810 inhibition of GSIS were also seen in isolated intact islets from human donors (n=4; one donor only).
**Fig. 26** illustrates the effect of knockdown of ABHD6 On GSIS. INS832/13 cells were either not transfected (stippled), transfected with PBS (white), transfected with two control siRNA (Control-siRNA-2 (dense dots) or Control-siRNA-5 (vertical lines) or two separate ABHD6-directed siRNAs (ABHD6-siRNA5 (horizontal lines) or ABHD6-siRNA6 (lighter dots)) and insulin secretion (as measured as mg protein/2hrs) was determined. Results indicate that inhibition of expression of ABHD6 significantly elevated glucose-stimulated insulin secretion. Basal insulin secretion was also increased with ABHD6-siRNA5, in comparison to different controls.

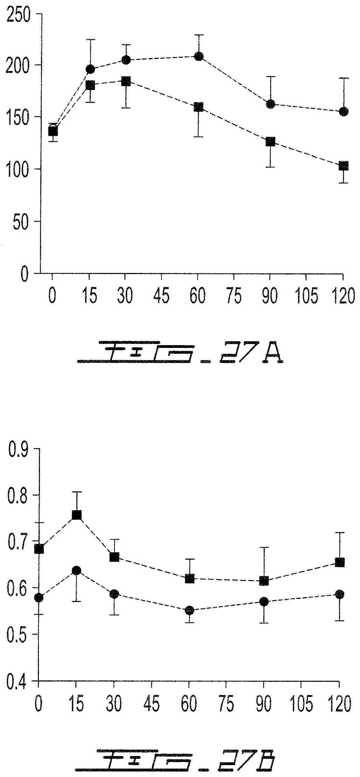

**Fig. 27** illustrates the effect of WWL70 in an oral glucose tolerance test. **(A)** Glycemia, as measured in mg/dL of blood, is determined in function of time (minutes) for vehicle-treated (●) or WWL70-treated (■) animals. **(B)** Insulin secretion, as measured in ng/mL of blood, is determined in function of time (minutes) for vehicle-treated (●) or WWL70-treated (■) animals.

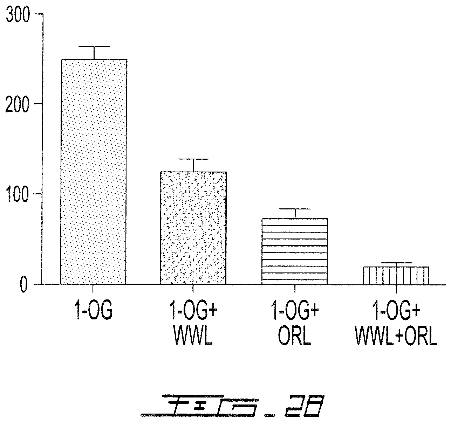

**Fig. 28** illustrated the measurement of MAG at the level of the cell membrane. Results are shown for pM of oleic acid in function of the treatment (control - 1-OG, WWL70 - 1-OG+WWL, orlistat - 1-OG+ORL or a combination of WWL70 and orlistat - 1-OG+WWL+ORL).

### DETAILED DESCRIPTION

There is provided herein a method for selecting agents useful for the promotion of insulin secretion (and eventually the treatment or the alleviation of symptoms associated with diabetes). These agents are selected in view of either being able to upregulate MAG level at the inner surface of the cyplasmic membrane of a cell and/or as being able to inhibit ABHD6 polypeptide activity or expression.

As it will be shown herein, the endocannabinoid 2-arachidonylglycerol (2-AG), which is a monoacylglycerol (MAG), takes part in controlling insulin secretion in the pancreatic β-cells. 2-AG is known to be involved in appetite control and act via the cannabinoid CB-1/2 receptors in the nervous system. CB-1/2 receptors are also found in peripheral tissues, including the pancreatic β-cell where they may participate in the control of IS. In obese hyperinsulinemic and insulin resistant individuals plasma levels of 2-AG are increased and likely contribute to orexigenic stimuli. Hypothalamic 2-AG levels are elevated in the genetically obese hyperinsulinemic ob/ob mouse. The possibility that the elevated 2-AG may contribute to the insulin secretion and hyperinsulinemia seen in obesity, was supported by the findings that in RINm5F β-cells, 2- AG levels rise in the presence of high glucose, suggesting that this MAG may have a role in GSIS. However, supra-physiological levels of 2-AG were found to inhibit GSIS.

There are two forms of MAG, i.e., 1- and 2-MAG. The role of GL/FFA cycle has recently been reviewed and it was suggested that, during the sequential hydrolysis of TG on the lipid droplets by ATGL and HSL, most (∼85%) of the MAG generated is 2-MAG. On the other hand, hydrolysis of lysophosphatidic acid (LPA) by lipid phosphate phosphatase (LPP) isoenzymes produces 1-MAG. 2-MAG can be converted to 1-MAG non-enzymatically under physiological conditions. Synthesis of 2-MAG (e.g., 2-AG) can also occur at the cell surface from receptor-stimulated hydrolysis of the phosphoinositides (PI) and phosphatidylcholine (PC) by phospholipase C, followed by the action of DAG lipases. Plasma membrane-bound sn1-DAG lipases hydrolyze membrane-bound sn1,2-DAG releasing 2-MAG, which has high proportion of unsaturated fatty acid. Interestingly, besides neuronal tissues, sn1-DAG lipases are expressed in significant levels in pancreatic β-cells. It is known that inhibition of β-cell plasma membrane DAG lipase activity by RHC80267 leads to lowered GSIS. It is important to note that MAG derived from lipolysis in the interior of the cell (i.e. away from cell membrane) is in a different compartment and may not have the same functional role as the MAG generated in the vicinity of the cell membrane. LPP enzymes are generally localized either in endoplasmic reticulum (ER) or cell membrane. The active site of ER-LPP is known to be facing towards the lumen, whereas, the cell membrane LPP has its active site facing extra-cellularly. Thus the product of LPP, 1-MAG, finds itself either in the ER-lumen or on the cell membrane outer surface, depending upon where it is generated. Intracellular levels of MAG are also regulated by MAG hydrolysis to glycerol and FFA. In the β-cells, there is very low expression of MAG lipase, which in other tissues viz., liver, adipose and brain is the major enzyme that hydrolyzes both 1- and 2-MAG. However, there are two other enzymes, α,β-domain containing hydrolase-6 (ABHD6) and ABHD12 that can hydrolyze MAG and are described in brain (Blankman et al.). ABHD6 expression was shown to be elevated in some cancer cells. Employing activity-based protein profiling and multidimensional protein identification technology (ABPP-MudPIT) approaches, it was shown that while ABHD6 is inhibited by WWL70 (Blankman et al.) with a Kᵢ of 70 nM (Li et al.), this compound as no effect on MAG lipase (MAGL) andABHD12 (Blankman et al.). Similarly, it was shown that JZL184 specifically inhibits MAGL and orlistat (tetrahydrolipstatin, which is pan-inhibitor of neutral glycerolipid lipases) inhibits ABHD12 with a Kᵢ of <100 nM. Expression level of ABHD6 or ABHD12 in comparison to MAGL, in β- cells is not known. Inasmuch as β-cells do conduct fuel-responsive lipolysis (measured as glycerol release), which is sensitive to inhibition by orlistat and also which is linked to insulin secretion, it appears that β-cells do have MAG hydrolysis activity. Since the expression level of MAGL in β-cells is very low, the MAG hydrolysis activity is likely to be due to ABHD6 and if so, then ABHD6 may also be linked to GSIS.

In the present application, it is shown that rodent and human β-cells have significant activity of ABHD6, an enzyme that controls monoacylglycerol (MAG) hydrolysis and the level of this metabolite near the cell membrane. Biochemical and pharmacological evidence are provided herewith and show that MAG produced in the vicinity of the plasma membrane acts as a signal for promoting exocytosis of insulin granules. This signaling pathway may also involve the activation of transient receptor potential vanilloid-1 (TRPV1 ) receptor by MAG, which triggers rapid influx of Ca²⁺ and insulin granule exocytosis. Specific inactivation of ABHD6 by the agent WWL70 leads to accumulation of MAG with an associated increase in insulin secretion. These results collectively show that inhibitors of ABHD6 have the potential to be developed as agents for the promotion of insulin secretion, for example, in a diabetic individual. These research findings implicate ABHD6 as a potential target for promoting insulin secretion.

The assay described herein is particularly useful to assess if agents have the ability to promote and/or increase insulin secretion from pancreatic β-cells. Such agents would be useful in the treatment of a condition associated with a lowered level of insulin secretion. As used herein, these conditions are commonly linked by the fact that the afflicted subject produces a lower plasma level of insulin than a healthy subject (e.g. normoglycemic), such that the afflicted subject become hyperglycemic. In these conditions, the pancreatic β-cells of the afflicted subject secrete less insulin that the pancreatic β-cells of the healthy subject. The normal blood glucose level in human is about 4 mM (4 mmol/L or 72 mg/dL) and can fluctuate between about 3.6 and 5.8 mM (mmol/L) throughout the day. As such, afflicted subject may have a blood glucose level that is higher than about 5.8 mM.

Insulin resistance is a condition in which body cells become less sensitive to the glucose-lowering effects of insulin. Insulin resistance in muscle and fat cells reduces glucose uptake (and so local storage of glucose as glycogen and triglycerides, respectively), whereas insulin resistance in liver cells results in reduced glycogen synthesis and storage and a failure to suppress glucose production and release into the blood. Insulin resistance normally refers to reduced glucose-lowering effects of insulin. However, other functions of insulin can also be affected. For example, insulin resistance in fat cells reduces the normal effects of insulin on lipids and results in reduced uptake of circulating lipids and increased hydrolysis of stored triglycerides. Increased mobilization of stored lipids in these cells elevates free fatty acids in the blood plasma. Elevated blood fatty-acid concentrations, reduced muscle glucose uptake, and increased liver glucose production all contribute to elevated blood glucose levels. If insulin resistance exists, more insulin needs to be secreted by the pancreas. If this compensatory increase does not occur, blood glucose concentrations increase and type II diabetes occurs. As such, the agents identified by the screening assay described herein could be useful in the treatment, alleviation of symptoms or prevention of insulin resistance.

One of the conditions associated with a lowered insulin level is diabetes. Diabetes can be divided into two broad type of diseases: type I and type II diabetes. Type II diabetes (also referred to as non-insulin-dependent diabetes mellitus (NIDDM), adult-onset diabetes or diabetes mellitus type II) is a disorder that is characterized by high blood glucose in the context of insulin resistance and relative insulin deficiency. Unlike type I diabetes, there is very little tendency toward ketoacidosis in type II diabetes. One effect that can occur is non-ketonic hyperglycemia which also is quite dangerous, though it must be treated very differently.

Another condition associated with a lowered level of insulin secretion is metabolic syndrome X. Metabolic Syndrome X is generally used to define a constellation of abnormalities that is associated with increased risk for the development of type II diabetes and atherosclerotic vascular disease. There is no consensus in the art as how to diagnose such a condition. Metabolic Syndrome X is can also be referred to, in the art, as "metabolic syndrome," "insulin resistance syndrome," and "syndrome X". Risk factors include, but are not limited to, central obesity, sedentary lifestyle, aging, diabetes mellitus, coronary heart disease and lipodystrophy. Related conditions and symptoms include, but are not limited to, fasting hyperglycemia (diabetes mellitus type II or impaired fasting glucose, impaired glucose tolerance, or insulin resistance), high blood pressure; central obesity (also known as visceral, male-pattern or apple-shaped adiposity), overweight with fat deposits mainly around the waist; decreased HDL cholesterol; elevated triglycerides. Associated diseases can also include hyperuricemia, fatty liver (especially in concurrent obesity) progressing to non-alcoholic fatty liver disease, polycystic ovarian syndrome (in women), and *acanthosis nigricans.*

As shown herein, the activity of ABHD6 is tightly linked to insulin secretion. The experimental data presented herewith elegantly shows that inhibition of ABHD6 activity favors insulin secretion. As such, the present application describes a method of characterizing an agent's ability for increasing insulin secretion in a subject. Those agents can be particularly useful for the treatment, alleviation of symptoms or prevention of diabetes (such as type II diabetes) or any other condition associated with a low level of insulin secretion/production (such as insulin resistance and metabolic syndrome X).

The assay described herein, in an embodiment, comprises the use of a reaction vessel having a ABHD6-based reagent. This reaction vessel is used to provide an environment for combining the agent and the ABHD6-reagent. In an embodiment, the agent that is being characterized is placed into the reaction vessel for a time sufficient to determine its effect on a parameter of the ABHD6-based reagent. As used herein, the ABHD6-based reagent is a biological entity that is derived from the ABHD6 polypeptide or its encoding nucleotide. The ABHD6-based reagent may be derived various sources such as, for example, humans (GenBank Accession No. NP_065727), mouse (GenBank Accession No: NP_079617), bovine (GenBank Accession No. NP_001068664), rat (GenBank Accession No. NP_001007681), frog (GenBank Accession No. NP_001086309), salmon (GenBank Accession No. NP_001133827).

*Polynucleotides encoding ABHD6.* In the assay provided herewith, a full length nucleotide sequence encoding the ABHD6 polypeptide or a fragment thereof can be used. A "fragment" of a ABHD6-encoding nucleotide sequence that encodes a biologically active portion (e.g. that retains ABHD6 specific lipase activity) of ADBH6 protein will encode at least 15, 25, 30, 50, 75, 100, 125, 150, 175, 200, 225, 250, 276, 300 or 325 contiguous amino acids, or up to the total number of amino acids present in a full-length ABHD6 polypeptide. Fragments of the ABHD6-encoding nucleotide sequence that are useful as specific hybridization probes and/or as specific PCR primers generally need not encode a biologically active portion of the ABHD6 polypeptide.

Nucleic acid molecules that are variants of the ABHD6-encoding nucleotide sequences disclosed herein can also be used. "Variants" of ABHD6 nucleotide sequences include those sequences that encode ABHD6 proteins disclosed herein but that differ conservatively because of the degeneracy of the genetic code. These naturally occurring allelic variants can be identified with the use of well-known molecular biology techniques, such as polymerase chain reaction (PCR) and hybridization techniques. Variant nucleotide sequences also include synthetically derived nucleotide sequences that have been generated, for example, by using site-directed mutagenesis but which still encode an ABHD6 protein having lipase activity. Generally, nucleotide sequence variants described herein will have at least 45%, 55%, 65%, 75%, 85%, 95%, or 98% identity to a particular nucleotide sequence disclosed herein. A variant ABHD6-encoding nucleotide sequence will encode an ABHD6 protein that has an amino acid sequence having at least 45%, 55%, 65%, 75%, 85%, 95%, or 98% identity to the amino acid sequence of the ABHD6 protein disclosed herein. It will be appreciated by those skilled in the art that DNA sequence polymorphisms that lead to changes in the amino acid sequences of ABHD6 proteins may exist within a population (e.g., the human population). Such genetic polymorphism in abhd6 gene may exist among individuals within a population due to natural allelic variation. Any and all such nucleotide variations and resulting amino acid polymorphisms or variations in abhd6 sequence that are the result of natural allelic variation and that do not alter the functional activity of ABHD6 proteins are intended to be used herein.

In addition to naturally-occurring allelic variants of ABHD6 sequences that may exist in the population, the skilled artisan will further appreciate that changes can be introduced by mutation into the nucleotide sequences described herein thereby leading to changes in the amino acid sequence of the encoded ABHD6 proteins, without altering the biological activity of the ABHD6 proteins. Such mutations can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

In the assay provided herewith, it is also possible to use the promoter of the abhd6 promoter operably linked to a reporter gene. The reporter gene can encode a protein that can be detected in the reaction vessel. The reporter gene can be, for example, the abhd6 gene itself or any other gene encoding a protein that can be detected in the reaction vessel (for example the green fluorescent protein or the β-galactosidase protein).

*ABHD6 polypeptide and related products.* The ADBH6-reagent maybe the full-length ABHD6 polypeptide or a biologically active fragment of the ABHD6 polypeptide that retains its characteristic lipase activity. "Fragments" or "biologically active portions" of the ABHD6 polypeptide include polypeptide fragments comprising amino acid sequences sufficiently identical to or derived from the amino acid sequence of the ABHD6 polypeptide and exhibiting at least one activity of the ABHD6 polypeptide (such as ABHD6-specific lipase activity), but which include fewer amino acids than the full-length ABHD6 polypeptide. Typically, biologically active portions comprise a domain or motif with at least one activity (such as lipase activity) of the ABHD6 polypeptide. A biologically active portion of the ABHD6 polypeptide can be a polypeptide that is, for example, 10, 25, 50, 100, 150, 200, 250, 300 or more amino acids in length. Such biologically active portions can be prepared by recombinant techniques and evaluated for one or more of the functional activities of a native ABHD6 polypeptide.

By "variants" is intended proteins or polypeptides having an amino acid sequence that is at least about 45%, 55%, 65%, preferably about 75%, 85%, 95%, or 98% identical to the ABHD6 polypeptide. Such variants generally retain the functional activity of the ABHD6 polypeptide. Variants include polypeptides that differ in amino acid sequence due to natural allelic variation or mutagenesis.

A biologically active portion of an ABHD6 protein or a variant of an ABHD6 protein does not need to have the level of lipase activity of the full-length ABHD6, but most retain at least some lipase activity (at least 50%, at least 60%, at least 70%, at least 80%, at least 90%, or even at least 95% of the lipase activity of the full-length ABHD6 lipase activity). A biological active portion or a variant of an ABHD6 protein does not need to have the specificity of the lipase activity of the full-length ABHD6, but is should retain at least some specificity to some of the targets of the full-length ABHD6 lipase. Preferably, the biologically active fragment or variant should be able to have some enzymatic activity towards 1-MAG and/or 2-MAG.

The methods described herein can also rely on a ABHD6 polypeptide chimeric or fusion proteins. As used herein, the "chimeric protein" or "fusion protein" comprises the ABHD6 polypeptide operably linked to a non-ABHD6 polypeptide. A "non-ABHD6 polypeptide" is intended to refer to a polypeptide having an amino acid sequence corresponding to a protein that is not substantially identical to the ABHD6 polypeptide, e.g., a protein that is different from the ABHD6 polypeptide and which is derived from the same or a different organism. Within the ABHD6 polypeptide fusion protein, the ABHD6 polypeptide can correspond to all or a portion of the ABHD6 polypeptide. The non-ABHD6 polypeptide can be fused to the N-terminus or C-terminus of the ABHD6 polypeptide.

*Reaction vessel.* The reaction vessel, where the agent is combined with the ABHD6-based reagent, can be an *in vitro* or *in vivo* environment. The contact between the agent and the ABHD6-based reagent must be made under conditions suitable and for a period of time that will enable the agent to interact with the ABHD6-based reagent and possible modify at least one of its parameters. Suitable *in vitro* environments can include, for example, a cell-free environment where a ABHD6 polypeptide, biologically active fragment thereof or a fusion protein comprising the ABHD6 polypeptide is combined in a reaction media comprising the appropriate reagents to enable the assessment of the biochemical lipase activity of the ABHD6 polypeptide or fragment thereof (buffers, substrates, additives, etc.).

Another suitable *in vitro* environment for the assay described herewith is a cultured cell. Such cell should be able to maintain viability in culture. The cultured cell should (i) express a polynucleotide encoding ABHD6 or a fragment thereof (ii) express a ABHD6-encoding polynucleotide or fragment thereof and/or (i) comprise the ABHD6 promoter region. In some instances, it may be advisable that the cell may also be able to secrete insulin. Such cell can be, for example, a primary cell line (such as, for example, primary pancreatic β-cells) or a cell line (such as, for example, INS 832/13, MIN6, INS1, A459, RINm5F or HIT, etc.). If a primary cell culture is used, the cell may be isolated (e.g. dissociated) from the pancreatic islets and/or preserved in a tissue-like structure, for example, as part of an intact (isolated) islet.

A further embodiment of the reaction vessel is a non-human animal (also referred to as an animal model). If the characterization of the agent occurs in an animal model, then the animal (such as a rodent) is administered with the agent. Various dosage and modes of administration maybe used to fully characterize the agent's ability to increase insulin secretion. The non-human animal can be, for example, a mouse (such as CD1-mouse or a C57BI6-mouse), a rat, a pig, monkey, etc.

Once the agent has been combined in the reaction vessel with the ABHD6-based reagent, a measurement or value of a parameter of the ABHD6-based reagent is made. This assessment may be made directly in the reaction vessel (by using a probe) or on a sample of such reaction vessel. The measurement of the parameter of the ABHD6-based reagent can be made either at the DNA level, the RNA level and/or the polypeptide level.

The measuring step can rely on the addition of a quantifier specific to the parameter to be assessed to the reaction vessel or a sample thereof. The quantifier can specifically bind to a parameter of a ABHD6-based reagent that is being assessed, such as, for example, a nucleotide product encoding ABHD6 or a ABHD6 polypeptide. In those instances, the amount of the quantifier that specifically bound (or that did not bind) to the ABHD6-based reagent will be determined to provide a measurement of the parameter of the ABHD6-based reagent. In another embodiment, the quantifier can be modified by a parameter of the ABHD6-based reagent, such as, for example, the ABHD6 lipase activity. In this specific instance, the amount of modified (or unmodified) quantifier will be determine to provide a measurement of the parameter of the ABHD6-based reagent. In an embodiment, the signal of the quantifier can be provided by a label that is either directly or indirectly linked to a quantifier.

Various parameters of the ABHD6-based reagent can be measured. For example, when the ABHD6-based reagent is a ABHD6 polypeptide or fragment thereof, the parameter that is measured can be the polypeptide lipase activity, the polypeptide quantity and/or stability. When the ABHD6-based reagent is a nucleotide encoding a ABHD6 polypeptide or fragment thereof, the parameter can be the level of expression or stability of the ABHD6-encoding nucleotide (e.g. ABHD6-encoding mRNA). Even though a single parameter is required to enable the characterization of the agent, it is also provided that more than one parameter of the ABHD6-based reagent may be measured.

If the measurement of the parameter is performed at the nucleotide level, then the transcription activity of the promoter associated with the ABHD6 gene can be assessed. This assessment can be made, for example, by placing a reporter vector in a cell. Such reporter vector includes the promoter region of the abhd6 gene (or fragment thereof) operably linked to a nucleotide encoding a reporter polypeptide (such as, for example, ABHD6, β-galactosidase, green-fluorescent protein, etc.). Upon the addition of the agent, the promotion of transcription from the promoter of the abhd6 gene is measured indirectly by measuring the transcription of the reporter polypeptide. In this particular embodiment, the quantifier is the reporter polypeptide and the signal associated to this quantifier that is being measured will vary upon the reporter polypeptide used. Alternatively or complementarily, the stability and/or the expression level of the ABHD6-encoding nucleotide can be assessed by quantifying the amount of a ABHD6-encoding nucleotide (for example using qPCR) or the stability of such nucleotide.

In one assay format, the expression of a nucleic acid encoding ABHD6 in a cell or tissue sample is monitored directly by hybridization to the nucleic acids specific for ABHD6. In another assay format, cell lines or tissues can be exposed to the agent to be tested under appropriate conditions and time, and total RNA or mRNA isolated, optionally amplified, and quantified.

If the measurement of the parameter is performed at the polypeptide level, an assessment of ABHD6 lipase activity can be performed. ABHD6 specifically hydrolyses 1-MAG and 2-MAG into glycerol and free fatty acids. As such, it is possible to assess ABHD6 activity by measuring the level of a reagent capable of being hydrolyzed by ABHD6. Such reagents include 1-MAG, 2-MAG or any other derivative of these compounds that can be labeled for ease of detection. Alternatively or complementarily, the level of the products of the enzymatic reaction wherein ABHD6 is implicated can be measured as well. For example, the reagents required for the enzymatic reaction can be labeled (for example with a radioactive or fluorescent tag) and the incorporation of that label into the resulting products (glycerol and free-fatty acids) can also be measured.

In another assay format, the specific activity of ABHD6, normalized to a standard unit, may be assayed in a cell-free system, a cell line or a cell population that has been exposed to the agent to be tested and compared to an unexposed control cell-free system, cell line or cell population.

Once the measurement has been made, it is extracted from the reaction vessel, and the value of the ABHD6-based reagent is compared to determine the presence or absence of inhibition by the agent of a parameter of the ABHD6-based reagent. In this comparison step, a comparison to a control value is made to determine the effect of the agent on the parameter of the ABHD6-based reagent. The control value may be the parameter of the ABHD6-based reagent in the absence of the agent. In this particular embodiment, the parameter of the ABHD6-reagent can be measured prior to the combination of the agent with the ABHD6-based reagent or in two replicates of the same reaction vessel where one of the screening system does not comprise the agent. The control value can also be the parameter of the ABHD6-based reagent in the presence of a control agent that is known not to increase/favor insulin secretion. Such control agent may be, for example, a pharmaceutically inert excipient (e.g. DMSO). The control value can also be the parameter of the ABHD6-based reagent obtained from a reaction vessel comprising cells or tissues from a healthy subject that does secretes a normal dose of insulin. The control value can also be a pre-determined value associated with a lack of increase in insulin secretion.

In an embodiment, the comparison can be made by an individual. In another embodiment, the comparison can be made in a comparison module. Such comparison module may comprise a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to determine the effect of the agent on the parameter of the ABHD6-based reagent with respect to the control value. An output of this comparison may be transmitted to a display device. The memory, accessible by the processor, receives and stores data, such as measured parameters of the ABHD6-based reagent or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

Once the comparison between the parameter of the ABHD6-based reagent and the control value is made, then it is possible to characterize the agent's ability to increase the insulin secretion of the subject. This characterization is possible because, as shown herein, an agent that limits the biological activity, expression or stability of the ABHD6 polypeptide or a ABHD6-encoding polynucleotide favors insulin secretion in pancreatic β-cells. As such, the characterization of the agent's ability to increase insulin secretion is based on that premise.

In an embodiment, the characterization can be made by an individual. In another embodiment, the characterization can be made with a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to characterize the ability of the agent with respect to insulin secretion based on the comparison of the value of the parameter of the ABHD6-based reagent with respect to the control value. The agent is characterized as being able to increase insulin secretion when the measurement of the parameter of the ABHD6-based reagent is lower than the control value. On the other hand, the agent is characterized as lacking the ability of increasing insulin secretion when the measurement of the parameter of the ABHD6-based reagent is higher than or equal to the control value. An output of this characterization may be transmitted to a display device. The memory, accessible by the processor, receives and stores data, such as measured parameters of the ABHD6-based reagent or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

The present application also describes a screening system for characterizing an agent's ability to increase insulin secretion in a subject. This screening system comprises a reaction vessel for combining the agent and the ABHD6-based reagent, a processor in a computer system, a memory accessible by the processor and an application coupled to the processor. The application or group of applications is(are) configured for receiving a value of a parameter of the ABHD6-based reagent in the presence of the agent; comparing the value of the parameter of the ABHD6-based reagent in the presence of the agent to a control value and/or characterizing the agent as having the ability to increase insulin secretion in the subject when the value of the parameter of the ABHD6-based reagent is lower than the control value or as lacking the ability to increase insulin secretion in the subject when the value of the parameter of the ABHD6-based reagent is equal to or higher than the control value.

The present application also describes a software product embodied on a computer readable medium. This software product comprises instructions for characterizing an agent's ability to increase insulin secretion in a subject. The software product comprises a receiving module for receiving a value of a parameter of a ABHD6-based reagent in the presence of the agent in a reaction vessel; a comparison module receiving input from the measuring module for determining if the value of the parameter of the ABHD6-based reagent in the presence of the agent is lower than, equal to or higher than a control value; a characterization module receiving input from the comparison module for identifying the usefulness of the agent for increasing insulin secretion. The agent is characterized as having the ability to increase insulin secretion in the subject is identified when the value of the parameter of the ABHD6-based reagent received from the comparison module is lower than the control value. On the other hand, the agent is characterized as lacking the ability to increase insulin secretion in the subject is identified when the value of the parameter of the ABHD6-based reagent received from the comparison module is equal to or higher than the control value. The comparison module and characterization module may each comprise a processor, a memory accessible by the processor to perform an application.

In an embodiment, an application found in the computer system of the screening system is used in the comparison module. A measuring module extracts/receives information from the reaction vessel with respect to the parameter of the ABHD6-based reagent. Such parameter may be the level biological activity of the ABHD6-based reagent (such as lipase activity), the level of expression and/or stability of the ABHD6-based reagent (polypeptide and/or mRNA). The receiving module is coupled to a comparison module which receives the value(s) of the parameter of the ABHD6-based parameter and determines if this value is lower than, equal to or higher than a control value. The comparison module can be coupled to a characterization module.

In another embodiment, an application found in the computer system of the screening system is used in the characterization module. The comparison module is coupled to the characterization module which receives the comparison and determines the agent's ability to increase insulin secretion based on this comparison. When the comparison indicates that the agent is capable of lowering the value of the parameter of the ABHD6-based reagent with respect to the control value, the agent is then characterized as being able to increase insulin secretion. When the comparison indicates that the agent is capable of augmenting or does not alter the value of the parameter of the ABHD6-based reagent with respect to the control value, the agent is then characterized as being unable to increase insulin secretion.

In a further embodiment, the receiving module, comparison module and characterization module are organized into a single discrete system. In another embodiment, each module is organized into different discrete system. In still a further embodiment, at least two modules are organized into a single discrete system.

As shown herein, the level of MAG is tightly linked to insulin secretion. The experimental data presented herewith elegantly shows that upregulation of MAG at the inner surface of a cytoplasmic member of a cell favors insulin secretion. As such, the present application describes a method of characterizing an agent's ability for increasing insulin secretion in a subject. Those agents can be particularly useful for the treatment, alleviation of symptoms or prevention of diabetes (such as type II diabetes) or any other condition associated with a low level of insulin secretion/production (such as insulin resistance and metabolic syndrome X).

The assay described herein comprises the use of a cell capable of producing a detectable level of MAG at the inner surface of its cytoplasmic membrane. The cell is then placed in a favorable environment for being combined with the agent. In an embodiment, the agent that is being characterized is combined with the cell for a time sufficient to determine its effect on a level of MAG at the inner surface of the cytoplasmic membrane. As used herein, monoacylglyceride or MAG comprises both 1-monoacylglyceride (1-MAG) and 2-monoacylglyceride (2-MAG) .

*Cell.* The cell to which the agent is combined can be an *in vitro* or *in vivo* environment. The contact between the agent and the cell reagent must be made under conditions suitable and for a period of time that will enable the agent to interact with the cell and possibly modify at least one of its parameters. Suitable *in vitro* environments can include, for example, a cell culture. Such cell should exhibit ABHD6 activity and be able to maintain viability in culture. Such cell can be, for example, a primary cell line (such as, for example, primary pancreatic β-cells) or a cell line (such as, for example, INS 832/13, MIN6, INS1, RINm5F, A459 or HIT, etc.). If a primary cell culture is used, the cell may be isolated from pancreatic islets or preserved in a tissue-like structure, for example, as part of an intact (isolated) islet. The cell can also be in a non-human animal (also referred to as an animal model). If the characterization of the agent occurs in an animal model, then the animal (such as a rodent) is administered with the agent.

Various dosage and modes of administration maybe used to fully characterize the agent's ability to increase insulin secretion. The non-human animal can be, for example, a mouse (such as CD1-mouse or a C57BI6-mouse), a rat, a pig, monkey, etc.

*Cell membrane.* The agent can also be combined with a cellular extract comprising a cell membrane or a portion thereof. The cellular extract should exhibit ABHD6 activity.

Once the agent has been combined with the cell or the cell membrane, a measurement or value of a level of MAG is made. This assessment may be made directly in the cell (by using imaging techniques or fluorescent sorting) or on a sample of such cell. MAG levels can also be made indirectly by measuring extracellular concentration of MAG, which is likely in equilibrium with the intracellular concentration of MAG.

The measuring step can rely on the addition of a quantifier specific to the parameter to be assessed to the cell or a sample thereof. The quantifier can be a label (such as a radioactive label or fluorescent label) that can be being transferred to newly synthesized MAG. In those instances, the amount of the label this is specifically integrated (or not) into new MAG molecules can be determined to provide a measurement of the level of MAG.

Once the measurement has been made, it is extracted from the cell, and the value of the level of MAG is compared to determine the presence or absence of modulation by the agent of a value of a MAG level. In this comparison step, a comparison to a control value is made to determine the effect of the agent on the value of the level of MAG. The control value may be the value of the level of MAG in the absence of the agent. In this particular embodiment, the MAG level can be measured prior to the combination of the agent to the cell or in two replicates where one of the screening system does not comprise the agent. The control value can also be the MAG level in the presence of a control agent that is known not to increase insulin secretion. Such control agent may be, for example, a pharmaceutically inert excipient (e.g. DMSO). The control value can also be the MAG level obtained from cells from a healthy subject that does secretes a normal dose of insulin. The control value can also be a pre-determined value of the level of MAG associated with a lack of increase in insulin secretion.

In an embodiment, the comparison can be made by an individual. In another embodiment, the comparison can be made in a comparison module. Such comparison module may comprise a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to determine the effect of the agent on the parameter of the MAG level with respect to the control value. An output of this comparison may be transmitted to a display device. The memory, accessible by the processor, receives and stores data, such as measured parameters of the MAG level or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

Once the comparison between the MAG level and the control value is made, then it is possible to characterize the agent's ability to increase the insulin secretion of the subject. This characterization is possible because, as shown herein, an agent that upregulates the MAG level at the inner surface of the cytoplasmic membrane favors insulin secretion in pancreatic β-cells. As such, the characterization of the agent's ability to increase insulin secretion is based on that premise.

In an embodiment, the characterization can be made by an individual. In another embodiment, the characterization can be made with a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to characterize the ability of the agent with respect to insulin secretion based on the comparison of the value of the MAG level with respect to the control value. The agent is characterized as being able to increase insulin secretion when the measurement of the MAG level is higher than the control value. On the other hand, the agent is characterized as lacking the ability of increasing insulin secretion when the measurement of the MAG level is lower than or equal to the control value. An output of this characterization may be transmitted to a display device. The memory, accessible by the processor, receives and stores data, such as measured parameters of MAG levels or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

The present application also describes a screening system for characterizing an agent's ability to increase insulin secretion in a subject. This screening system comprises a cell for combining the agent, a processor in a computer system, a memory accessible by the processor and an application coupled to the processor. The application or group of applications is(are) configured for receiving a value of a level of MAG in the presence of the agent; comparing the value of the level of MAG in the presence of the agent to a control value and/or characterizing the agent as having the ability to increase insulin secretion in the subject when the value of the level of MAG is higher than the control value or as lacking the ability to increase insulin secretion in the subject when the value of the level of MAG is equal to or lower than the control value.

The present application also describes a software product embodied on a computer readable medium. This software product comprises instructions for characterizing an agent's ability to increase insulin secretion in a subject. The software product comprises a receiving module for receiving a value of a level of MAG in the presence of the agent in a cell; a comparison module receiving input from the measuring module for determining if the value of the level of MAG in the presence of the agent is lower than, equal to or higher than a control value; a characterization module receiving input from the comparison module for identifying the usefulness of the agent for increasing insulin secretion. The agent is characterized as having the ability to increase insulin secretion in the subject is identified when the value of the level of MAG received from the comparison module is higher than the control value. On the other hand, the agent is characterized as lacking the ability to increase insulin secretion in the subject is identified when the value of the level of MAG received from the comparison module is equal to or higher than the control value. The comparison module and characterization module may each comprise a processor, a memory accessible by the processor to perform an application.

In an embodiment, an application found in the computer system of the screening system is used in the comparison module. A measuring module extracts/receives information from the cell with respect to the level of MAG. The receiving module is coupled to a comparison module which receives the value(s) of the level of MAG and determines if this value is lower than, equal to or higher than a control value. The comparison module can be coupled to a characterization module.

In another embodiment, an application found in the computer system of the screening system is used in the characterization module. The comparison module is coupled to the characterization module which receives the comparison and determines the agent's ability to increase insulin secretion based on this comparison. When the comparison indicates that the agent is capable of augmenting the value of the level of MAG with respect to the control value, the agent is then characterized as being able to increase insulin secretion. When the comparison indicates that the agent is capable of lowering or does not alter the value of the parameter of the ABHD6-based reagent with respect to the control value, the agent is then characterized as being unable to increase insulin secretion.

In a further embodiment, the receiving module, comparison module and characterization module are organized into a single discrete system. In another embodiment, each module is organized into different discrete system. In still a further embodiment, at least two modules are organized into a single discrete system.

The present application also describes a method of stimulating insulin secretion and ultimately treat or alleviate the symptoms of diabetes (preferably type II diabetes) and/or metabolic syndrome X. Since the downregulation of ABHD6 activity has been shown to be useful in increasing insulin secretion, it is believed that the administration of genetic-based therapy will be useful in the treatment of diabetes and/or metabolic syndrome X. In order to do so, in an embodiment, an agent capable of downregulating or repressing the expression of the abhd6 gene and/or the stability of a transcript of this gene is administered to the individual.

*Antisense.* In a particular embodiment, an antisense nucleic acid or oligonucleotide is wholly or partially complementary to, and can hybridize with, an abhd6 nucleic acid (either DNA or RNA) having the sequence or a fraction thereof of the abhd6 gene or its corresponding mRNA or cDNA. For example, an antisense nucleic acid or oligonucleotide comprising 10, 15 or even 20 nucleotides can be sufficient to limit and even inhibit expression of the abdh6 gene or its transcript. Alternatively, an antisense nucleic acid or oligonucleotide can be complementary to 5' or 3' untranslated regions, or can overlap the translation initiation codon (5' untranslated and translated regions) of the abhd6 gene. In another embodiment, the antisense nucleic acid is wholly or partially complementary to, and can hybridize with, a target nucleic acid that encodes an ABHD6 protein. As non-limiting examples, antisense oligonucleotides may be targeted to hybridize to the following regions: mRNA cap region; translation initiation site; translational termination site; transcription initiation site; transcription termination site; polyadenylation signal; 3' untranslated region; 5' untranslated region; 5' coding region; mid coding region; 3' coding region; DNA replication initiation and elongation sites. Preferably, the complementary oligonucleotide is designed to hybridize to the most unique 5' sequence of the abdh6 gene, including any of about 15-35 nucleotides spanning the 5' coding sequence. The antisense oligonucleotide can be synthesized, formulated as a pharmaceutical composition, and administered to a subject.

*Triplex oligonucleotides.* In addition, oligonucleotides can be constructed which will bind to duplex nucleic acid (i.e., DNA:DNA or DNA:RNA), to form a stable triple helix containing or triplex nucleic acid. Such triplex oligonucleotides can inhibit transcription and/or expression of the abdh6 gene or its transcript. Triplex oligonucleotides are constructed using the base-pairing rules of triple helix formation and the nucleotide sequence of the abdh6 gene.

*Oligonucleotides.* In the context of this application, the term "oligonucleotide" refers to naturally-occurring species or synthetic species formed from naturally-occurring subunits or their close homologs. The term may also refer to moieties that function similarly to oligonucleotides, but have non-naturally-occurring portions. Thus, oligonucleotides may have altered sugar moieties or inter-sugar linkages. Exemplary among these are phosphorothioate and other sulfur containing species which are known in the art. In preferred embodiments, at least one of the phosphodiester bonds of the oligonucleotide has been substituted with a structure that functions to enhance the ability of the compositions to penetrate into the region of cells where the RNA whose activity is to be modulated is located. It is preferred that such substitutions comprise phosphorothioate bonds, methyl phosphonate bonds, or short chain alkyl or cycloalkyl structures. In accordance with other preferred embodiments, the phosphodiester bonds are substituted with structures which are, at once, substantially non-ionic and non-chiral, or with structures which are chiral and enantiomerically specific. Persons of ordinary skill in the art will be able to select other linkages for use in the practice of the methods described herein. Oligonucleotides may also include species that include at least some modified base forms. Thus, purines and pyrimidines other than those normally found in nature may be so employed. Similarly, modifications on the furanosyl portions of the nucleotide subunits may also be affected. Examples of such modifications are 2'-O-alkyl- and 2'-halogen-substituted nucleotides. Some non-limiting examples of modifications at the 2' position of sugar moieties which are useful include OH, SH, SCH₃, F, OCH₃, OCN, O(CH₂), NH₂ and O(CH₂)nCH₃, where n is from 1 to about 10. Such oligonucleotides are functionally interchangeable with natural oligonucleotides or synthesized oligonucleotides, which have one or more differences from the natural structure. All such analogs are comprehended by this embodiment so long as they function effectively to hybridize with the abhd6 gene DNA or its corresponding RNA (or cDNA) to inhibit the function thereof.

*Expression vectors.* Alternatively, expression vectors derived from retroviruses, adenovirus, herpes or vaccinia viruses or from various bacterial plasmids may be used for delivery of nucleotide sequences to the targeted organ (e.g. pancreas), tissue or cell population. Methods which are well known to those skilled in the art can be used to construct recombinant vectors which will express nucleic acid sequence presented herewith.

*RNAi.* RNA interference (RNAi) is a post-transcriptional gene silencing process that is induced by a miRNA or a dsRNA (a small interfering RNA or siRNA), and has been used to modulate gene expression. Generally, RNAi is being performed by contacting cells with a double stranded siRNA ou a small hairpin RNA (shRNA). However, manipulation of RNA outside of cells is tedious due to the sensitivity of RNA to degradation. It is thus also encompassed herein a deoxyribonucleic acid (DNA) compositions encoding small interfering RNA (siRNA) molecules, or intermediate siRNA molecules (such as shRNA), comprising one strand of an siRNA. Accordingly, the present application describes an isolated DNA molecule, which includes an expressible template nucleotide sequence of at least about 16 nucleotides encoding an intermediate siRNA, which, when a component of an siRNA, mediates RNA interference (RNAi) of a target RNA. The present application further describes the use of RNA interference (RNAi) to modulate the expression of the abdh6 gene. While this method is not limited to a particular mode of action, RNAi may involve degradation of messenger RNA by an RNA induced silencing complex (RISC), preventing translation of the transcribed targeted mRNA. Alternatively, it may involve methylation of genomic DNA, which shuts down transcription of a targeted gene. The suppression of gene expression caused by RNAi may be transient or it may be more stable, even permanent.

*Small interfering RNA (siRNA).* This method refers to any nucleic acid molecule capable of mediating RNA interference "RNAi" or gene silencing. For example, siRNA can be double stranded RNA molecules from about ten to about 30 nucleotides long that are named for their ability to specifically interfere with protein expression. In one embodiment, the siRNAs are 12-28 nucleotides long, more preferably 15-25 nucleotides long, even more preferably 19-23 nucleotides long and most preferably 21-23 nucleotides long. Therefore preferred siRNA are 12, 13, 14, 15, 16, 17, 18, 19, 20, 21, 22, 23, 24, 25, 26, 27, 28 nucleotides in length. As used herein, siRNA molecules need not to be limited to those molecules containing only RNA, but further encompass chemically modified nucleotides and non-nucleotides. siRNA are herein designed to decrease expression of the abhd6 gene in a target cell (e.g. a pancreatic β-cell) by RNA interference. siRNAs comprise a sense region and an antisense region wherein the antisense region comprises a sequence complementary to an mRNA sequence for the abdh6 gene and the sense region comprises a sequence complementary to the antisense sequence of the abdh6 gene's mRNA. An siRNA molecule can be assembled from two nucleic acid fragments wherein one fragment comprises the sense region and the second fragment comprises the antisense region of siRNA molecule. The sense region and antisense region can also be covalently connected via a linker molecule. The linker molecule can be a polynucleotide linker or a non-polynucleotide linker.

*Ribozymes.* A ribozyme (from ribonucleic acid enzyme, also called RNA enzyme or catalytic RNA) is an RNA molecule that catalyzes a chemical reaction. Some ribozymes may play an important role as therapeutic agents, as enzymes which target defined RNA sequences, as biosensors, and for applications in functional genomics and gene discovery. Ribozymes can be genetically engineered to specifically cleave a transcript of an abhd6 gene.

*Gene therapy.* Delivery of the gene or genetic material into the cell is the first critical step in gene therapy treatment of a disorder. A large number of delivery methods are well known to those of skill in the art. Preferably, the nucleic acids are administered for *in vivo* or *ex vivo* gene therapy uses. Non-viral vector delivery systems include DNA plasmids, naked nucleic acid, and nucleic acid complexed with a delivery vehicle such as a liposome. Viral vector delivery systems include DNA and RNA viruses, which have either episomal or integrated genomes after delivery to the cell.

The use of RNA or DNA based viral systems for the delivery of nucleic acids take advantage of highly evolved processes for targeting a virus to specific cells in the body and trafficking the viral payload to the nucleus. Viral vectors can be administered directly to patients *(in vivo)* or they can be used to treat cells *in vitro* and the modified cells then administered to patients *(ex vivo).* Conventional viral based systems for the delivery of nucleic acids could include retroviral, lentiviral, adenoviral, adeno-associated and herpes simplex virus vectors for gene transfer. Viral vectors are currently the most efficient and versatile method of gene transfer in target cells and tissues. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

In applications where transient expression of the nucleic acid is preferred, adenoviral based systems are typically used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors are also used to transduce cells with target nucleic acids, e.g., in the in vitro production of nucleic acids and peptides, and for in vivo and ex vivo gene therapy procedures.

In particular, numerous viral vector approaches are currently available for gene transfer in clinical trials, with retroviral vectors by far the most frequently used system. All of these viral vectors utilize approaches that involve complementation of defective vectors by genes inserted into helper cell lines to generate the transducing agent. pLASN and MFG-S are examples are retroviral vectors that have been used in clinical trials.

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system.

Replication-deficient recombinant adenoviral vectors (Ad) are predominantly used in transient expression gene therapy; because they can be produced at high titer and they readily infect a number of different cell types. Most adenovirus vectors are engineered such that a transgene replaces the Ad E1a, E1b, and E3 genes; subsequently the replication defective vector is propagated in human 293 cells that supply the deleted gene function in trans. Ad vectors can transduce multiple types of tissues *in vivo,* including non-dividing, differentiated cells such as those found in the liver, kidney and muscle tissues. Conventional Ad vectors have a large carrying capacity.

In many gene therapy applications, it is desirable that the gene therapy vector be delivered with a high degree of specificity to a particular tissue type (such as the pancreas). A viral vector is typically modified to have specificity for a given cell type by expressing a ligand as a fusion protein with a viral coat protein on the viruses outer surface. The ligand is chosen to have affinity for a receptor known to be present on the cell type of interest.

Gene therapy vectors can be delivered *in vivo* by administration to an individual subject, typically by systemic administration (e.g., intravenous, intraperitoneal, intramuscular, subdermal, or intracranial infusion) or topical application. Alternatively, vectors can be delivered to cells *ex vivo,* such as cells explanted from an individual patient (e.g., pancreatic islet cells or even β-cells) or universal donor hematopoietic stem cells, followed by re-implantation of the cells into the subject, usually after selection for cells which have incorporated the vector.

*Ex vivo* cell transfection for gene therapy (e.g. via re-infusion of the transfected cells into the host organism) is well known to those of skill in the art. In a preferred embodiment, cells are isolated from the subject organism, a nucleic acid (gene or cDNA) of interest is introduced therein, and the cells are re-infused back into the subject organism (e.g., patient). Various cell types suitable for *ex vivo* treatment are well known to those of skill in the art. In one embodiment, stem cells are used in *ex vivo* procedures for cell transfection and gene therapy. The advantage to using stem cells is that they can be differentiated into other cell types in vitro, or can be introduced into a mammal (such as the donor of the cells) where they will engraft at an appropriate location (such as in the bone marrow). Methods for differentiating CD34+ cells *in vitro* into clinically important immune cell types using cytokines such as for example GM-CSF, IFN-γ and TNF-α are known. Stem cells are isolated for transduction and differentiation using known methods. For example, stem cells can be isolated from bone marrow cells by panning the bone marrow cells with antibodies which bind unwanted cells, such as CD4+ and CD8+ (T cells), CD45+ (panB cells), GR-1 (granulocytes), and lad (differentiated antigen presenting cells).

The present invention will be more readily understood by referring to the following examples which are given to illustrate the invention rather than to limit its scope.

### EXAMPLE I - MATERIALS AND METHODS

*Materials.* Cell culture supplies were from Corning (Corning, NY) and Fisherbrand (Canada). 2-Arachidonylglycerol was from Sigma Chemicals, WWL70 was obtained from Cayman Chemical Company and AMG9810 was from Tocris Bioscience and dissolved in dimethylsulfoxide (DMSO) before their use in insulin secretion experiments. D-[U-¹⁴C]glucose was from GE Healthcare (Canada) and palmitate sodium salt was from Nu-Check Prep (Elysian, MN). Bicinchoninic acid protein assay from Pierce (Rockford, IL) was used. Stock unlabelled palmitate was prepared at 4 mM in 5% defatted BSA as described elsewhere (Roduit et al.). Orlistat was purchased from Sigma and JZL184 was from Cayman. Antibodies were obtained from Abcam.

*Cell culture.* INS 832/13 cells (Hohmeir et al.) were cultured at 37°C in a humidified atmosphere containing 5% CO₂ in RPMI 1640 with sodium bicarbonate, supplemented with 10% (v/v) fetal calf serum (Wisent), 10 mM HEPES, pH 7.4, 2 mM L-glutamine, 1 mM sodium pyruvate and 50 µM β-mercaptoethanol (complete RPMI). Cells were grown to 80% confluence. Media were changed to RPMI 1640 containing 3 mM glucose supplemented as the complete RPMI 24 h prior to the experiments. Insulin secretion incubations were conducted in Krebs-Ringer bicarbonate buffer containing 10 mM HEPES, pH 7.4 (KRBH).

*Islet isolation.* All procedures were approved by the Institutional Committee for the Protection of Animals at the Centre Hospitalier de l'Université de Montreal Research Center. Wistar rats or C57BI6 mice or CD1 mice (all male) from Charles River (St-Constant, QC, Canada) were anaesthetized with Somnotol® (MTC Pharmaceuticals, Canada) and sacrificed by exsanguination. Pancreatic islets were isolated by collagenase (type XI from Sigma) digestion of total pancreas (Gotoh et al.), followed by separation (centrifugation at 1040 x *g)* on a Histopaque™ 1119, 1077 (Sigma) gradient. Isolated islets were handpicked and cultured overnight in a petri dish at 37°C in a humidified atmosphere containing 5% CO₂ in RPMI 1640 with sodium bicarbonate, supplemented with 10% fetal calf serum, 10 mM HEPES pH 7.4, 2 mM L-glutamine, 1 mM sodium pyruvate, 100 U/mL penicillin and 100 µg/mL streptomycin.

Human islets were obtained from the Human Islet Isolation Core of MDRC, following appropriate ethical guidelines. The donor was a male without any known metabolic disease. Islets were hand-picked and cultured overnight before use. Both rodent and human islets were also treated with trypsin to disperse islet cells and then cultured overnight in RPMI complete medium. These overnight cultured dispersed islets cells were then used for examining the effect of various pharmacological agents.

*Insulin secretion measurement.* INS 832/13 cells were washed in KRBH containing 1 mM glucose and 0.5% defatted BSA (KRBH 1G/0.5%BSA) and pre-incubated for 45 min in KRBH 1G/0.5%BSA in presence of pharmacological agents (at indicated concentrations) or vehicle (DMSO). For examining the effect of WWL70 (an inhibitor of ABHD6), AMG9810 (an antagonist of TRPV1-recepor), orlistat (lipase inhibitor) and/or JZL184 (monoacylglycerol lipase inhibitor) were added first in pre-incubation media and then during incubation. When examining the effect of 2-AG, it was added to cells at 0 to 1 M concentration at 2 mM and 10 mM glucose. Insulin secretion from INS 832/13 cells was measured from either 45-min or 2-h static incubations in KRBH containing various glucose concentrations, 0.5% defatted BSA and pharmacological agents or vehicle, with or without 35 mM KCI or 0.3 mM palmitate, as specified.

For insulin secretion from rat, mouse or human islets, batches of 10 islets were washed in KRBH containing 2.8 mM glucose and 0.5% defatted BSA, and pre-incubated for 45 min in KRBH containing 2.8 mM glucose, 0.5% defatted BSA and different pharmacological agents (at indicated concentrations) or DMSO. Islets were then incubated for either 45 min or 2 h in KRBH containing various glucose concentrations or 2.8 mM glucose plus 35 mM KCI, 0.5% defatted BSA in the presence or absence of drugs (at indicated concentrations) or vehicle (DMSO). At the end of the incubation, media were collected and insulin extracted from cells or islets in acid-ethanol (1.5% HCl, 75% ethanol). Total insulin contents and media insulin concentrations were determined by radioimmunoassay using human insulin standards (Linco Research, MO).

*Effect of Lipase inhibitors on [U*-*¹⁴C]*-*glucose incorporation into lipids.* INS 832/13 cells were incubated overnight at 11 mM [U-¹⁴C]-glucose in RPMI medium in a CO₂-incubator in order to pre-label all the lipids in the cells. Then the cells were washed in KRBH (1 mM [U-¹⁴C]-glucose) and pre-incubated for 45 min in KRBH at 1 mM [U-¹⁴C]-glucose to bring metabolism to basal level. After this, cells were further incubated for 2 h in KRBH at 1 and 10 mM [U-¹⁴C]-glucose and the indicated inhibitors. Panlipase inhibitor, orlistat at 50 µM, WWL70 at 10 µM and JZL184 at 1 µM were used, where shown. Then the cells were washed rapidly and flash-frozen in liquid nitrogen and extracted for lipids. Lipids were separated by TLC and the associated radioactivity was quantified. Neutral lipids were separated on boric acid/silica gel-TLC, with two solvent systems, first petroleum ether: diethyl ether: acetic acid (70:30:1), followed by second solvent system up to half of the plates, chloroform: acetone: acetic acid (60:40:1). Phospholipids were separated with a solvent system, chloroform: methanol: water (65:25:4).

*Lipolysis determination.* INS 832/13 cells were washed in KRBH 1G/0.5%BSA and pre-incubated for 45 min in KRBH 1G/0.5%BSA. All incubation conditions and inhibitors were as described for insulin secretions. Glycerol release, an index of lipolysis, was determined by a coupled enzymatic assay (Peyot et al.).

*Quantitative real-time RT-PCR.* Total RNA was extracted from INS 832/13 cells, islets and rodent tissues using the Rneasy Mini kit™ (Qiagen) with RNase-free DNase (Qiagen). RNA (3 µg) was reverse transcribed to cDNA using M-MLV reverse transcriptase (Invitrogen, Canada) and hexamers as previously described (Delghingaro-Augusto et al.). Gene expression was determined by the standard curve method and normalized to the expression of β-actin. Real-time PCR analysis was performed using the Rotor-Gene R3000™ (Corbett Research, Australia) and the LCR Faststart DNA masterplus SYBR Green™ reagent (Roche, Canada). Primers for MAG lipase, ABHD6 and TRPV1-receptor were designed using Primer3™ software. Results are expressed as the ratio of target mRNA to β-actin mRNA.

*Immunoblot analysis.* INS 832/13 cells, islets and rodent tissues were processed for SDS-PAGE and Western blotting. Cells or tissues were washed with cold PBS and lysed using a lysis buffer containing 20 mM Tris-HCl pH 7.5, 150 mM NaCl, 1 mM EDTA, 1 mM EGTA, 1% Triton X-100, 0.1% SDS, protease inhibitors, 1 mM Na₃VO₄ and 2.5 mM Na₄P₂O₇. Lysates were sonicated, aliquots were taken for protein assay and samples were stored at -80°C. Proteins from total cell extracts (20 µg protein) were separated on 8% SDS-PAGE and transferred to nitrocellulose membranes (Scheicher & Schuell, Germany) for Western blotting. Blotted proteins were probed using monoclonal antibodies (Abcam) against MAG lipase and ABHD6 according to suppliers' protocols. Horseradish peroxidase-conjugated goat anti-mouse IgG (Bio-Rad, Hercules, CA) was used as second antibody with SuperSignal West Pico™ chemiluminescence (Pierce) for detection.

*Cytosolic Ca²⁺ measurement in rat islets.* Ca²⁺ was measured by adapting a procedure as previously described (Jahanshasi et al.). Briefly, dispersed rat islet cells were plated on a 42 mm coverslip and incubated overnight at 37°C in complete medium at 11 mM glucose. Cells were starved in RPMI complete medium at 2.8 mM glucose for 2 h and loaded in KRBH containing 2.8 mM glucose, 1% BSA (defatted), 2.5 mM probenicid, 0.2 mM sulfinpyrazone and 6 µM Fluo-4AM™ + pluronic F-127™ (1:1) (Invitrogen, USA) for 75 min at 37 C. Coverslips were then mounted in a closed perifusion chamber (Germany) and cells perifused with KRBH containing 1% BSA, 2.5 mM probenicid, 0.2 mM sulfinpyrazone and 2.8 mM glucose or 16.7 mM glucose or 2.8 mM glucose + 35 mM KCI using a syringe pump (New Era pump system). The microscope, perifusion chamber and solutions were maintained at 37 C. Cells were incubated for 180 s at 2.8 mM glucose and one image was recorded for baseline determination. Images were then recorded every second for a period of 10 min in presence of 16.7 mM glucose, and for a period of 2 min in presence of KCI using a Leica TCS SP5™ inverted confocal microscope. Incubation with 16.7 mM glucose was started at time 20 s, but the analyses were started at 150 s to exclude baseline fluctuations (caused by the autofocus stabilization device of the microscope scanner and false-positive fluorescence due to osmolarity changes in the perifusion chamber). Inhibitor of ABHD6 (WWL70, 10 M) and of TRPV1-R (AMG9810, 10 M) were included in to the perifusion medium at both the glucose concentrations. Perfusions were conducted sequentially, glucose alone, then with WWL70 followed by AMG9810, in the same perfusion chamber.

*RNAi-knockdown of ABHD6 in INS832*/*13 cells.* INS 832/13 cells were plated at a density of 150 000 cells/ well in a 12-well plate, in 1 ml complete RPMI medium, one day prior to RNAi transfection. On the next day, RNAi duplexes and lipofectamine-RNAimax™ were prepared separately. Then 24 pmol of each RNAi duplex was diluted in 100 µl Opti-MEM™ without serum and mixed gently. And at the same time, 2 µl of lipofectamine-RNAimax was diluted in 100 µl Opti-MEM without serum and mixed gently. Then the diluted RNAi duplex and RNAi-max reagents were mixed gently (200 µl volume) and incubated at room temperature for 20 min. This 200 µl mixture (which gives a final concentration of 20nmol of RNAi), prepared separately for each well, was then added to the corresponding well with approximately 30% confluent INS cells. Following RNAi transfection, cells were incubated for 16 to 72 h. in a CO₂ incubator at 37°C. Then the cells were starved by incubation in RPMI-1640 with 10% FBS and 1 mM glucose for 2 h. followed by washing one time with KRBH (containing 1 mM glucose and 0.5% BSA) and pre-incubation with the same KRBH for 45 min. Finally for following insulin secretion, cells were incubated in the KRBH buffer with 1 mM or 10 mM glucose for 2 h. The media and the cells were collected for insulin and protein assay as described above. The ABHD6-directed siRNAs and the control siRNAs were obtained from Ambion (Catalog #4390771; CGGAAAUUGUUUUUGGAAAtt (ABHD6-6 sense or SEQ ID NO: 9), UUUCCAAAAACAAUUUCCGgt (ABHD6-6 anti-sense or SEQ ID NO: 10), CAGUUUGUAGAAUGCCUUAtt (ABHD6-5 sense or SEQ ID NO: 11), UAAGGCAUUCUACAAACUGat (ABHD6-5 anti-sense or SEQ ID NO: 12)). ABHD6-5 sense and antisense oligos are first annealed (i.e., hybridized) and then used as ABHD6-siRNA-5, as indicated in Fig. 26. ABHD6-6 sense and antisense oligos are used in a similar fashion. Control siRNAs, which are non-targeting siRNAs with limited sequence similarity to known genes were obtained from Ambion (Catalog #AM4613 and AM4642) and were used to transfect the cells as described above. Additionally, non-treated cells and pBS (plasmid vector Bluescript) transfected cells were used as controls as well. Results of insulin secretion and ABHD6 expression in ABHD6-siRNA transfected cells were compared with different controls.

*Statistical analysis.* Values are expressed as means ± SEM. Statistical analysis was performed using one-way ANOVA with Dunnett's post-test for multiple comparisons or two-way ANOVA with Bonferroni's post-test for multiple comparisons using GraphPad Prism™ (GraphPad Software).

### EXAMPLE II - INHIBITION OF ABHD6 ACTIVITY FAVORS GLUCOSE STIMULATED INSULIN SECRETION

The materials and methods used in the example are presented in Example I.

*Effect of 2-AG on GSIS in INS 832*/*13 β -cells:* In INS 832/13 β-cells insulin secretion is augmented by 2-AG at both basal (2mM) and high (10 mM) glucose (Fig. 1) and interestingly GSIS at high glucose concentration and arachidonic acid, is accompanied by enhanced [1-¹⁴C]-arachidonic acid incorporation in to 1-MAG and 2-AG (Fig. 2). The pan-lipase inhibitor orlistat, which bocks GL/FFA cycling, curtails insulin secretion (Fig. 3) and [1-¹⁴C]-arachidonic acid incorporation into 1-AG and 2-AG by inhibiting lipolysis. It was noticed that micromolar concentration of 2-AG restores orlistat-inhibited insulin secretion (Fig. 3). These results suggested that orlistat inhibition of insulin secretion was probably related to the reduced formation and availability of MAG (2-AG) as the supplementation of 2-AG re-established insulin secretion and also indicated the possibility that MAG is a signaling metabolite, which mediates insulin secretion.

*Expression profile of MAG lipases in various tissues and β -cells:* Results shown in Fig. 4 indicate that while MAG lipase expression at mRNA level is very low in β-cells and in islets from rat, mouse and human, its protein expression in western blots is noticeable in islets but weak or no detectable signal in cultured cells such as A549, INS 832/13 or MIN6 cells. However MAGL is expressed in significant amount in rat adipose, liver and brain. Expression of ABHD6, the cytosol-facing membrane-bound MAG hydrolytic enzyme, both at mRNA and protein level, is significant in islets and INS cells, although comparatively liver expressed more of this protein (Fig. 5 & 6). However, expression of ABHD12, the exterior-facing membrane bound MAG hydrolytic enzyme, is low in INS cells and rodent islets even though human islets showed much higher level of this enzyme (data not shown).

*Effect of MAG hydrolysis inhibition on [14C]-glucose incorporation into various lipids:* In order to further examine whether MAG is indeed playing a role in signaling insulin secretion, the effect of specific inhibitors of MAG lipase (JZL184) and ABHD6 (WWL70) and also pan-lipase inhibitor, orlistat on MAG accumulation on insulin secretion in INS 832/13 β-cells was tested. Under conditions where all the internal lipid carbons are labeled by [¹⁴C]-glucose, incubation with 10 mM glucose led to increased synthesis of all lipid fractions including phospholipids, triglycerides, DAG and 1- and 2-MAG and also the amount of ¹⁴C-FFA released into the medium (Figs. 7-12). Results indicate that none of the lipase inhibitors had any significant effect on glucose incorporation in phospholipids (Fig. 7). As expected, TG accumulated in the presence of orlistat (Fig. 8). MAGL inhibitor, JZL184 did not have any effect (Fig. 13) while ABHD6 inhibition only had marginal effect on TG accumulation (Fig. 8). Neither of the inhibitors had any effect on total DAG levels (including both 1,2- and 2,3-isomers). These results suggest that DAG is in rapid equilibrium with MAG and TG (Fig. 9 & 13). 2-MAG is formed mostly by the hydrolysis of DAG. Interestingly, while orlistat showed no effect on 2-MAG levels, inhibition of either MAG-lipase (JZL184) or ABHD6 (WWL70) caused an elevation of this lipid (Fig. 10 & 13). If DAG and MAG are in rapid equilibrium, it is possible that high concentration of orlistat (50 µM) used in this experiment, could have maintained the steady-state level of 2-MAG, by inhibiting both its formation and degradation. On the other hand, orlistat significantly reduced the formation of 1-MAG, while inhibitors of MAGL and ABHD6 caused an increase (Fig. 11 & 13). Considering that significant amount of the 1-MAG arises from the hydrolysis of LPA by LPP isoenzymes, that are not known to be inhibited by orlistat, it is surprising that orlistat lowered the production of 1-MAG. It is also important to consider that the intra-cellular location of MAG (either 1-MAG or 2-MAG) accumulation of may vary depending upon the enzyme that is inhibited, i.e., MAGL or ABHD6. This may have relevance for the signaling functional ability of the accumulating MAG in the cell. This became a significant possibility when it was noticed that the efflux of labeled FFA from INS 832/13cells, is strongly decreased by WWL70-inhibition of ABHD6 (Fig. 12), whereas, MAGL inhibition (by JZL184) had no effect (Fig. 13), even though both the inhibitors lead to the build up of MAG. Thus, the FFA generated by ABHD6 hydrolysis of MAG, beneath the surface of the plasma membrane is more readily available for export, while the MAGL-generated FFA is not, suggesting that MAGL and the MAG that accumulates due to MAGL inhibition are probably located in the interior of cytosol, away from plasma membrane.

*Stimulation of GSIS by specific inhibition of ABHD6:* It was then examined if inhibition of MAG hydrolysis in β-cells influences insulin secretion. While MAGL inhibition had no significant effect on GSIS (Fig. 14), inhibition of ABHD6 almost doubled the GSIS at 5 and 10 mM glucose (Fig. 15). It appears that only glucose-responsive insulin secretion is stimulated by WWL70, as the portion of GSIS amplified by palmitate, is not further enhanced by this inhibitor (Fig. 15). There was no further additive or synergistic effect on GSIS by the combination of WWL70 and JZL184. As noticed in earlier studies, orlistat strongly inhibited GSIS without or with palmitate (Fig. 15). Thus only the MAG that accumulates when ABHD6 is inhibited but not when MAGL is inhibited, is capable of promoting enhanced glucose-responsive insulin secretion in INS 832/13 β-cells. At both intermediate (5 mM) and high (10 mM) glucose concentrations, WWL70 showed a dose-dependent enhancement of GSIS, reaching near maximal effect at 10 µM concentration (Fig. 16). Exposure of INS 832/13 cells to either of the inhibitors for a total of over 3.5 h during pre-incubation and incubation, had no significant effect on the total insulin content of the cells. Inhibition of ABDH6 by RNAi was shown to increase insulin secrete in cultured cells (Fig. 26).

### EXAMPLE III - INTERACTION BETWEEN ABHD6 AND TRPV1-RECEPTOR IN GLUCOSE STIMULATED INSULIN SECRETION

The materials and methods used in the example are presented in Example I.

*TRPV1-receptor is involved in the WWL70-enhanced GSIS:* TRPV1 receptor, also known as vanilloid receptor is activated by various agents including H⁺ ions, anandamide, capsaicin, heat etc. The presence of TRPV1-receptor in pancreatic β-cells has been investigated by different groups and while its presence and functionality has been shown in normal rat islet β-cells, studies done on ZDF rats and in the Type-1 diabetes model, NOD mice (8 week old with insulinitis) were unable to show the TRPV1-R presence in the islets. It has been demonstrated that TRPV1-R has its ligand/agonist binding site intracellularly and its activation by capsaicin or other agonists lead to Ca²⁺ influx, which is a pre-requisite for insulin granule fusion with plasma membrane for exocytosis. Because of the uncertainty regarding TRPV1-R localization in β-cells, a systematic screening of islets and INS 832/13 β-cells employing qRT-PCR and also western blot analysis was conducted in the same samples. As shown in Fig. 17, the expression of TRPV1-R at both mRNA (Fig 17B) as well as protein (Fig. 17A) level in tissues, islets and β-cell lines. Activation of TRPV1-R by capsaicin both *in vitro* and also *in vivo* has been shown to elevate insulin secretion and Ca²⁺ influx. Both 1- and 2-MAG have recently been shown to activate TRPV1-R *in vitro* leading to Ca²⁺ influx and also *in vivo.* Since it is shown herein that TRPV1-R is expressed in islets and β-cells, it was tested whether this receptor is involved in the regulation of GSIS and its augmentation by ABHD6 inhibition by employing a highly specific TRPV1-R antagonist, AMG9810. It was noticed that AMG9810 dose-dependently decreased GSIS marginally but significantly in INS cells at intermediate and high glucose concentrations (Fig. 18). However, its inhibition is more marked under conditions when GSIS is enhanced by WWL70 (Fig. 19). Presence or absence of palmitate during GSIS incubations had no influence on AMG9810 inhibition. It is important to note that AMG9810 does not inhibit GSIS completely, even at high concentrations, which may suggest that the TRPV1-R in β-cells only partially contributes to the Ca²⁺ influx needed for exocytosis. It has been suggested that in islet β-cells, while the first phase insulin secretion is coupled to L-type Ca²⁺ channels, the second phase secretion is probably coupled to R-type Ca²⁺ channels.

*Influence of WWL70 and AMG9810 on KCI-stimulated and amino acid stimulated insulin secretion:* At low glucose concentration (1 mM), a large excess of KCI (35 mM) leads to plasma membrane depolarization and Ca²⁺ influx, which promotes the fusion of insulin granules that are already docked at the membrane. Thus, this process bypasses the need for either K⁺-ATP channels or other receptors for Ca²⁺ influx. Neither WWL70 nor AMG9810 had any effect on KCI-stimulated insulin secretion in INS 832/13 cells (Fig. 20). This supports the notion that the MAG that accumulates due to WWL70 inhibition of ABHD6, is needed for TRPV1-R activation for subsequent Ca²⁺ influx and insulin secretion and high KCI bypasses this mechanism. However, stimulation of insulin secretion by glutamine and leucine at low glucose, is also enhanced by WWL70, similar to GSIS and is also inhibited by AMG9810 (Fig. 20). Thus it is likely that the amino acid-stimulated insulin secretion also involves the activation of TRPV1-receptor by MAG. No significant insulin secretion was noticed in response to arginine in INS 832/13 cells.

*Effect of WWL70 in isolated primary pancreatic islets and dispersed islet cells:* Similar to INS 832/13 cells, ABHD6 inhibition in isolated intact rat islets (Fig. 21) and dispersed rat islet cells (Fig. 22) from Wistar rats, by WWL70 leads to elevated GSIS in static incubations and this enhancement is antagonized by AMG9810. Qualitatively very similar results were obtained using intact islets from two strains of mice, CD1 (Fig. 23) and C57BI6 (Fig. 24) and in intact human islets (Fig. 25), obtained from a disease-free donor, even though AMG9810 inhibited only WWL70-augmented GSIS in both mouse and human islets without significant effect on GSIS *per se,* unlike in INS cells and rat islets. Thus it appears that TRPV1-R mediated Ca²⁺ influx upon MAG activation may not be an obligatory pathway of GSIS in mouse and human islets, although this mechanism does boost insulin secretion significantly above what is achieved by the basic GSIS mechanisms. However, it is possible that MAG formation may be obligatory for GSIS, inasmuch as WWL70, which causes MAG accumulation, enhances insulin secretion. It has been reported that TRPV1-KO mice (C57BI6J genetic background) are relatively resistant to high-fat diet induced obesity and also that these mice exhibit much better glucose tolerance and insulin sensitivity. Also TRPV1-KO mice have reduced insulinemia unlike their controls, which have the tendency to show poor glucose tolerance. However, these effects were attributed primarily to the loss of TRPV1 in peripheral neurons in the islets. Considering that activation of TRPV1-R by its ligands capsaicin and resiniferatoxin *in vivo* elevates plasma insulin levels and that deletion of TRPV1-R reduces plasma insulin along with the present observations that TRPV1-R is indeed expressed in islets and β-cells and has a role in insulin secretion *in vitro* and *ex vivo,* strongly indicate that this receptor in β-cells may have an important function *in vivo* to regulate insulin secretion. Thus collectively our results support the concept that ABHD6 is an important regulator of GSIS and its selective inhibition can cause MAG to accumulate and activate TRPV1-R thereby promoting insulin secretion. Therefore, compounds that selectively target ABHD6 have the potential to be developed as diabetes therapeutics, which increase circulating insulin levels. Such drugs may also have the potential to increase insulin sensitivity in the peripheral tissues by TRPV1-R activation, as administration of capsaicin was shown to decrease insulin resistance.

### EXAMPLE IV - IN VIVO ENHANCEMENT OF INSULIN SENSITIVITY OF ABHD6 INHIBITOR

Normal CD1 mice were fasted for 4 h prior to the oral glucose tolerance test (OGTT). WWL70 (10mg/kg of body weight) was given intraperitoneally to 6 mice as a microsuspension in mannitol:PEG300:Tween80 (20:1:1), 2h prior to glucose load. Control mice (6) were given only vehicle. After 2h, all mice were given a oral gavage of glucose (2 g/kg of body weight), followed by blood collection from the tail vein at the indicated times. Blood glucose (glucometer; Accu-Check™, Roche Applied Science) and plasma insulin (ELISA) (insulin mouse ultrasensitive electroimmunoassay, ALPCO Diagnostics, Salem, NH) were measured. Administration of WWL70 better controlled the glycemia (Fig. 27A) and significantly elevated the plasma insulin levels (Fig. 27B). The lower glycemia (Fig 27A) observed even at 2h (i.e., 4 h after WWL70 injection) is suggestive of enhanced peripheral insulin sensitivity in these mice.

### EXAMPLE V - MEASUREMENT OF MAG AT THE CELL MEMBRANE

8 µg of INS832/13 cell membrane extracts were incubated with 50 µM of MAG (1-oleoylglycerol) and the indicated inhibitors for 1 h at 30°C. Released oleic acid was measured by HPLC after Dole's extraction and derivatization with bromoacetaminophenone. As shown in Fig. 28, in comparison with the control (1-OG), the ABHD6 inhibitor, WWL70 (1-OG+WWL), or the pan-lipase inhibitor (1-OG+ORL) inhibited the 1-OG hydrolysis activity. The combination of WWL and orlistat (10G+WWL+ORL) abolished the 1-OG hydrolysis activity completely. Similar incubations with cytosolic fraction of the INS cells yielded 10-fold less 1-OG hydrolytic activity (not shown) as compared to the membrane fraction, indicating that MAG (1-OG, here) hydrolysis in INS -cells is predominantly conducted by ABHD6, present in the plasma membrane.

### REFERENCES

Blankman JL, Simon GM, Cravatt BF: A comprehensive profile of brain enzymes that hydrolyze the endocannabinoid 2-arachidonoylglycerol. Chem Biol 14:1347-1356, 2007.
Delghingaro-Augusto V, Nolan CJ, Gupta D, Jetton TL, Latour MG, Peshavaria M, Madiraju SR, Joly E, Peyot ML, Prentki M, Leahy J: Islet beta cell failure in the 60% pancreatectomised obese hyperlipidaemic Zucker fatty rat: severe dysfunction with altered glycerolipid metabolism without steatosis or a falling beta cell mass. Diabetologia 52:1122-1132, 2009.
Gotoh M, Maki T, Satomi S, Porter J, Bonner-Weir S, O'Hara CJ, Monaco AP: Reproducible high yield of rat islets by stationary in vitro digestion following pancreatic ductal or portal venous collagenase injection. Transplantation 43:725-730, 1987.
Hohmeier HE, Mulder H, Chen G, Henkel-Rieger R, Prentki M, Newgard CB: Isolation of INS-1-derived cell lines with robust ATP-sensitive K+ channel-dependent and - independent glucose-stimulated insulin secretion. Diabetes 49:424-430, 2000.
Jahanshahi P, Wu R, Carter JD, Nunemaker CS: Evidence of diminished glucose stimulation and endoplasmic reticulum function in nonoscillatory pancreatic islets. Endocrinology 150:607-615, 2009.
Li W, Blankman JL, Cravatt BF: A functional proteomic strategy to discover inhibitors for uncharacterized hydrolases. J Am Chem Soc 129:9594-9595, 2007.
Peyot ML, Nolan CJ, Soni K, Joly E, Lussier R, Corkey BE, Wang SP, Mitchell GA, Prentki M: Hormone-sensitive lipase has a role in lipid signaling for insulin secretion but is nonessential for the incretin action of glucagon-like peptide 1. Diabetes 53:1733-1742,2004.
Roduit R, Nolan C, Alarcon C, Moore P, Barbeau A, Delghingaro-Augusto V, Przybykowski E, Morin J, Masse F, Massie B, Ruderman N, Rhodes C, Poitout V, Prentki M: A role for the malonyl-CoA/long-chain acyl-CoA pathway of lipid signaling in the regulation of insulin secretion in response to both fuel and nonfuel stimuli. Diabetes 53:1007-1019, 2004.

## Claims

1. A method of characterizing an agent's ability to increase insulin secretion in a subject, said method comprising:
(a) extracting a value of a parameter of the ABHD6-based reagent in the presence of the agent, wherein the value is obtained from combining the agent with a ABHD6- based reagent in a reaction vessel;
(b) comparing the value of the parameter of the ABHD6-based reagent of step (a) to a control value; and
(c) characterizing the agent as:
i. having the ability to increase insulin secretion in the subject when the value of the parameter of the ABHD6-based reagent of step (a) is lower than the control value; and
ii. lacking the ability to increase insulin secretion in the subject when the value of the parameter of the ABHD6-based reagent of step (a) is equal to or higher than the control value;
wherein the ABHD6-based reagent is a ABHD6 polypeptide and the parameter is the ABHD6 polypeptide lipase activity, quantity or stability; or
wherein the ABHD6-based reagent is a polynucleotide encoding the ABHD6 polypeptide and the parameter is the level of expression or stability of the polynucleotide.

2. The method of claim 1, wherein the ABHD6-based reagent is the ABHD6 polypeptide and the parameter is the ABHD6 lipase activity.

3. The method of claim 1, wherein the ABHD6-based reagent is the polynucleotide encoding the ABHD6 polypeptide and the parameter is the level of expression of the polynucleotide.

4. The method of any one of claims 1 to 3, wherein said combining comprises adding the agent to a cell having the ABHD6-based reagent.

5. The method of any one of claims 1 to 4, wherein the control value is at least one of:
the parameter of the ABHD6-based reagent in the absence of the agent, the parameter of the ABHD6-based reagent in the presence of a control agent that fails to increase insulin secretion in the subject and a pre-determined value associated with a lack of increase of insulin secretion.

6. The method of any one of claims 1 to 5, wherein the subject is suffering from at least one of the following conditions: diabetes, preferably type II diabetes, and metabolic syndrome X.

7. A screening system for characterizing an agent's ability to increase insulin secretion in a subject, said screening system comprising:
(a) a reaction vessel for combining the agent with a ABHD6-based reagent;
(b) a processor in a computer system;
(c) a memory accessible by the processor, wherein the processor is configured to perform a computer-based method to carry out the steps of:
i. receiving a value of a parameter of the ABHD6-based reagent extracted from the ABHD6-based reagent in the presence of the agent;
ii. comparing the value of the parameter of the ABHD6-based reagent of step (i) to a control value; and
iii. characterizing the agent as having the ability to increase insulin secretion in the subject when the value of the parameter of the ABHD6-based reagent is lower than the control value and as lacking the ability to increase insulin secretion in the subject when the value of the parameter of the ABHD6-based reagent is equal to or higher than the control value;
wherein the ABHD6-based reagent is a ABHD6 polypeptide and the parameter is the ABHD6 polypeptide lipase activity, quantity or stability; or
wherein the ABHD6-based reagent is a polynucleotide encoding the ABHD6 polypeptide and the parameter is the level of expression or stability of the polynucleotide.

8. The screening system of claim 7, wherein the ABHD6-based reagent is the ABHD6 polypeptide and the parameter is the ABHD6 lipase activity.

9. The screening system of claim 7, wherein the ABHD6-based reagent is the polynucleotide encoding a ABHD6 polypeptide and the parameter is the level of expression of the polynucleotide.

10. The screening system of any one of claims 7 to 9, wherein the reaction vessel comprises a cell having the ABHD6-based reagent.

11. The screening system of any one of claims 7 to 10, wherein the control value is at least one of: the parameter of the ABHD6-based reagent in the absence of the agent, the parameter of the ABHD6-based reagent in the presence of a control agent that fails to increase insulin secretion in the subject and a pre-determined value associated with a lack of increase of insulin secretion.

12. The screening system of any one of claims 7 to 11, wherein the subject is suffering from at least one of the following conditions: diabetes, preferably type II diabetes, and metabolic syndrome X.

13. A software product embodied on a computer readable medium and comprising instructions for characterizing an agent's ability to increase insulin secretion in a subject, said product comprising:
(a) a receiving module for receiving a value of a parameter of a ABHD6-based reagent in the presence of the agent in a reaction vessel;
(b) a comparison module for determining if the value of the parameter of the ABHD6-based reagent in the presence of the agent is lower than, equal to or higher than a control value and generating a corresponding output;
(c) a characterization module receiving the corresponding output from the comparison module and adapted to characterize the usefulness of the agent for increasing insulin secretion, wherein:
i. the agent is characterized as having the ability to increase insulin secretion in the subject when the value of the parameter of the ABHD6-based reagent is lower than the control value; and
ii. the agent is characterized as lacking the ability to increase insulin secretion in the subject when the value of the parameter of the ABHD6-based reagent is equal to or higher than the control value;
wherein the ABHD6-based reagent is a ABHD6 polypeptide and the parameter is the ABHD6 polypeptide lipase activity, quantity or stability; or
wherein the ABHD6-based reagent is a polynucleotide encoding the ABHD6 polypeptide and the parameter is the level of expression or stability of the polynucleotide.

14. The software product of claim 13, wherein the ABHD6-based reagent is the ABHD6 polypeptide and the parameter is the ABHD6 lipase activity.

15. The software product of claim 13, wherein the ABHD6-based reagent is the polynucleotide encoding a ABHD6 polypeptide and the parameter is the level of expression of the polynucleotide.

16. The software product of any one of claims 13 to 15, wherein the reaction vessel comprises a cell having the ABHD6-based reagent.

17. The software product of any one of claims 13 to 16, wherein the control value is at least one of: the parameter of the ABHD6-based reagent in the absence of the agent, the parameter of the ABHD6-based reagent in the presence of a control agent that fails to increase insulin secretion in the subject and a pre-determined value associated with a lack of increase of insulin secretion.

18. The software product of any one of claims 13 to 17, wherein the subject is suffering from at least one of the following conditions: diabetes, preferably type II diabetes, and metabolic syndrome X.

## Patentansprüche

1. Verfahren zur Kennzeichnung der Fähigkeit eines Wirkstoffs, eine Insulinsekretion in einem Subjekt zu erhöhen, wobei das Verfahren umfasst:
(a) Nehmen eines Wertes eines Parameters des ABHD6-basierten Reagenzes in der Anwesenheit des Wirkstoffs, wobei der Wert durch Zusammenbringen des Wirkstoffs mit einem ABHD6-basierten Reagenz in einem Reaktionsgefäß erhalten wurde,
(b) Vergleichen des Wertes des Parameters des ABHD6-basierten Reagenzes aus Schritt (a) mit einem Kontrollwert, und
(c) Kennzeichnen des Wirkstoffs durch:
i. Vorhandensein der Fähigkeit zur Erhöhung einer Insulinsekretion in dem Subjekt, wenn der Wert des Parameters des ABHD6-basierten Reagenzes aus Schritt (a) kleiner ist als der Kontrollwert, und
ii. Fehlen der Fähigkeit zur Erhöhung einer Insulinsekretion in dem Subjekt, wenn der Wert des Parameters des ABHD6-basierten Reagenzes aus Schritt (a) gleich oder größer ist als der Kontrollwert,
wobei das ABHD6-basierte Reagenz ein ABHD6-Polypeptid ist und der Parameter die ABHD6-Polypeptid-Lipaseaktivität, -menge oder -stabilität ist,
oder
wobei das ABHD6-basierte Reagenz ein Polynukleotid kodierend das ABHD6-Polypeptid ist und der Parameter das Niveau einer Expression oder einer Stabilität des Polynukleotids ist.

2. Verfahren nach Anspruch 1, wobei das ABHD6-basierte Reagenz das ABHD6-Polypeptid ist und der Parameter die ABHD6-Lipaseaktivität ist.

3. Verfahren nach Anspruch 1, wobei das ABHD6-basierte Reagenz das Polynukleotid kodierend das ABHD6-Polypeptid ist und der Parameter das Niveau einer Expression des Polynukleotids ist.

4. Verfahren nach einem der Ansprüche 1 bis 3, wobei das Zusammenbringen ein Hinzufügen des Wirkstoffs zu einer Zelle umfasst, welche das ABHD6-basierte Reagenz aufweist.

5. Verfahren nach einem der Ansprüche 1 bis 4, wobei der Kontrollwert wenigstens einer ist aus: dem Parameter des ABHD6-basierten Reagenzes in der Abwesenheit des Wirkstoffs, dem Parameter des ABHD6-basierten Reagenzes in der Anwesenheit eines Kontrollwirkstoffs, welcher eine Insulinsekretion in dem Subjekt nicht erhöhen kann, und einem vorbestimmten Wert, welcher mit dem Fehlen eines Anstiegs einer Insulinsekretion assoziiert ist.

6. Verfahren nach einem der Ansprüche 1 bis 5, wobei das Subjekt an wenigstens einer der folgenden Erkrankungen leidet: Diabetes, vorzugsweise Typ II Diabetes, und metabolisches Syndrom X.

7. Screeningssystem zur Kennzeichnung der Fähigkeit eines Wirkstoffs, eine Insulinsekretion in einem Subjekt zu erhöhen, wobei das Screeningssystem umfasst:
(a) ein Reaktionsgefäß zum Zusammenbringen des Wirkstoffs mit einem ABHD6-basierten Reagenz,
(b) einen Prozessor in einem Computersystem,
(c) einen Speicher, auf welchen der Prozessor zugreifen kann, wobei der Prozessor konfiguriert ist, um ein computerbasiertes Verfahren ausführen zu können, um die Schritte auszuführen:
i. Empfangen eines Wertes eines Parameters des ABHD6-basierten Reagenzes, welcher von dem ABHD6-basierten Reagenz in der Anwesenheit des Wirkstoffs genommen wurde,
ii. Vergleichen des Wertes des Parameters des ABHD6-basierten Reagenzes aus Schritt (i) mit einem Kontrollwert, und
iii. Kennzeichnen des Wirkstoffs durch Vorhandensein der Fähigkeit zur Erhöhung einer Insulinsekretion in dem Subjekt, wenn der Wert des Parameters des ABHD6-basierten Reagenzes kleiner ist als der Kontrollwert, und durch Fehlen der Fähigkeit zur Erhöhung einer Insulinsekretion in dem Subjekt, wenn der Wert des Parameters des ABHD6-basierten Reagenzes gleich oder größer ist als der Kontrollwert,
wobei das ABHD6-basierte Reagenz ein ABHD6-Polypeptid ist und der Parameter die ABHD6-Polypeptid-Lipaseaktivität, -menge oder -stabilität ist, oder
wobei das ABHD6-basierte Reagenz ein Polynukleotid kodierend das ABHD6-Polypeptid ist und der Parameter das Niveau einer Expression oder einer Stabilität des Polynukleotids ist.

8. Screeningsystem nach Anspruch 7, wobei das ABHD6-basierte Reagenz das ABHD6-Polypeptid ist und der Parameter die ABHD6-Lipaseaktivität ist.

9. Screeningsystem nach Anspruch 7, wobei das ABHD6-basierte Reagenz das Polynukleotid kodierend ein ABHD6-Polypeptid ist und der Parameter das Niveau einer Expression des Polynukleotids ist.

10. Screeningssystem nach einem der Ansprüche 7 bis 9, wobei das Reaktionsgefäß eine Zelle umfasst, welche das ABHD6-basierte Reagenz aufweist.

11. Screeningsystem nach einem der Ansprüche 7 bis 10, wobei der Kontrollwert wenigstens einer ist aus: dem Parameter des ABHD6-basierten Reagenzes in der Abwesenheit des Wirkstoffs, dem Parameter des ABHD6-basierten Reagenzes in der Anwesenheit eines Kontrollwirkstoffs, welcher eine Insulinsekretion in dem Subjekt nicht erhöhen kann, und einem vorbestimmten Wert, welcher mit dem Fehlen eines Anstiegs einer Insulinsekretion assoziiert ist.

12. Screeningsystem nach einem der Ansprüche 7 bis 11, wobei das Subjekt an wenigstens einer der folgenden Erkrankungen leidet: Diabetes, vorzugsweise Typ II Diabetes, und metabolisches Syndrom X.

13. Softwareprodukt, verkörpert auf einem computerlesbaren Medium und umfassend Instruktionen zur Kennzeichnung der Fähigkeit eines Wirkstoffs, eine Insulinsekretion in einem Subjekt zu erhöhen, wobei das Produkt umfasst:
(a) ein Empfangsmodul zum Empfangen eines Wertes eines Parameters eines ABHD6-basierten Reagenzes in der Anwesenheit des ABHD6-basierten Reagenzes in einem Reaktionsgefäß,
(b) ein Vergleichsmodul zur Bestimmung, ob der Wert des Parameters des ABHD6-basierten Reagenzes in der Anwesenheit des Wirkstoffs kleiner, gleich oder größer als ein Kontrollwert ist, und zur Erzeugung einer entsprechenden Ausgabe,
(c) ein Kennzeichnungsmodul, welches die entsprechende Ausgabe vom Vergleichsmodul empfängt und so angepasst ist, dass es die Eignung des Wirkstoffs zur Erhöhung einer Insulinsekretion kennzeichnet, wobei
i. der Wirkstoff gekennzeichnet wird, dass er die Fähigkeit zur Erhöhung einer Insulinsekretion in dem Subjekt aufweist, wenn der Wert des Parameters des ABHD6-basierten Reagenzes kleiner ist als der Kontrollwert,
ii. der Wirkstoff gekennzeichnet wird, dass er keine Fähigkeit zur Erhöhung einer Insulinsekretion in dem Subjekt aufweist, wenn der Wert des Parameters des ABHD6-basierten Reagenzes gleich oder größer ist als der Kontrollwert,
wobei das ABHD6-basierte Reagenz ein ABHD6-Polypeptid ist und der Parameter die ABHD6-Polypeptid-Lipaseaktivität, -menge oder -stabilität ist, oder
wobei das ABHD6-basierte Reagenz ein Polynukleotid kodierend das ABHD6-Polypeptid ist und der Parameter das Niveau einer Expression oder einer Stabilität des Polynukleotids ist.

14. Softwareprodukt nach Anspruch 13, wobei das ABHD6-basierte Reagenz das ABHD6-Polypeptid ist und der Parameter die ABHD6-Lipaseaktivität ist.

15. Softwareprodukt nach Anspruch 13, wobei das ABHD6-basierte Reagenz das Polynukleotid kodierend ein ABHD6-Polypeptid ist und der Parameter das Niveau einer Expression des Polynukleotids ist.

16. Softwareprodukt nach einem der Ansprüche 13 bis 15, wobei das Reaktionsgefäß eine Zelle umfasst, welche das ABHD6-basierte Reagenz aufweist.

17. Softwareprodukt nach einem der Ansprüche 13 bis 16, wobei der Kontrollwert wenigstens einer ist aus: dem Parameter des ABHD6-basierten Reagenzes in der Abwesenheit des Wirkstoffs, dem Parameter des ABHD6-basierten Reagenzes in der Anwesenheit eines Kontrollwirkstoffs, welcher eine Insulinsekretion in dem Subjekt nicht erhöhen kann, und einem vorbestimmten Wert, welcher mit dem Fehlen eines Anstiegs einer Insulinsekretion assoziiert ist.

18. Softwareprodukt nach einem der Ansprüche 13 bis 17, wobei das Subjekt an wenigstens einer der folgenden Erkrankungen leidet: Diabetes, vorzugsweise Typ II Diabetes, und metabolisches Syndrom X.

## Revendications

1. Procédé de caractérisation de la capacité d'un agent à augmenter la sécrétion d'insuline chez un sujet, ledit procédé comprenant :
(a) l'extraction d'une valeur d'un paramètre du réactif à base de ABHD6 en présence de l'agent, la valeur étant obtenue par combinaison de l'agent avec un réactif à base de ABHD6 dans une cuve de réaction ;
(b) la comparaison de la valeur du paramètre du réactif à base de ABHD6 de l'étape (a) à une valeur témoin ; et
(c) la caractérisation de l'agent comme :
i. ayant la capacité à augmenter la sécrétion d'insuline chez le sujet lorsque la valeur du paramètre du réactif à base de ABHD6 de l'étape (a) est inférieure à la valeur témoin ; et
ii. ne possédant pas la capacité à augmenter la sécrétion d'insuline chez le sujet lorsque la valeur du paramètre du réactif à base de ABHD6 de l'étape (a) est égale ou supérieure à la valeur témoin ;
dans lequel le réactif à base de ABHD6 est un polypeptide ABHD6 et le paramètre est l'activité lipase, la quantité ou la stabilité du polypeptide ABHD6 ; ou
dans lequel le réactif à base de ABHD6 est un polynucléotide codant pour le polypeptide ABHD6 et le paramètre est le taux d'expression ou la stabilité du polynucléotide.

2. Procédé de la revendication 1, dans lequel le réactif à base de ABHD6 est le polypeptide ABHD6 et le paramètre est l'activité lipase de ABHD6.

3. Procédé de la revendication 1, dans lequel le réactif à base de ABHD6 est le polynucléotide codant pour le polypeptide ABHD6 et le paramètre est le taux d'expression du polynucléotide.

4. Procédé de l'une quelconque des revendications 1 à 3, dans lequel ladite combinaison comprend l'ajout de l'agent à une cellule ayant le réactif à base de ABHD6.

5. Procédé de l'une quelconque des revendications 1 à 4, dans lequel la valeur témoin est au moins l'un de : le paramètre du réactif à base de ABHD6 en l'absence de l'agent, le paramètre du réactif à base de ABHD6 en présence d'un agent témoin qui ne produit pas d'augmentation de sécrétion d'insuline chez le sujet et une valeur prédéterminée associée à une absence d'augmentation de la sécrétion d'insuline.

6. Procédé de l'une quelconque des revendications 1 à 5, dans lequel le sujet souffre d'au moins une des affections suivantes : le diabète, de préférence le diabète de type II, et le syndrome métabolique X.

7. Système de criblage pour caractériser la capacité d'un agent à augmenter la sécrétion d'insuline chez un sujet, ledit système de criblage comprenant :
(a) une cuve de réaction pour combiner l'agent avec un réactif à base de ABHD6 ;
(b) un processeur dans un système informatique ;
(c) une mémoire accessible par le processeur, le processeur étant configuré pour conduire un procédé informatisé pour conduire les étapes de :
i. réception d'une valeur d'un paramètre du réactif à base de ABHD6 extrait du réactif à base de ABHD6 en présence de l'agent ;
ii. comparaison de la valeur du paramètre du réactif à base de ABHD6 de l'étape (i) à une valeur témoin ; et
iii. caractérisation de l'agent comme ayant la capacité à augmenter la sécrétion d'insuline chez le sujet lorsque la valeur du paramètre du réactif à base de ABHD6 est inférieure à la valeur témoin et comme ne possédant pas la capacité à augmenter la sécrétion d'insuline chez le sujet lorsque la valeur du paramètre du réactif à base de ABHD6 est égal ou supérieure à la valeur témoin ;
dans lequel le réactif à base de ABHD6 est un polypeptide ABHD6 et le paramètre est l'activité lipase, la quantité ou la stabilité du polypeptide ABHD6 ; ou
dans lequel le réactif à base de ABHD6 est un polynucléotide codant pour le polypeptide ABHD6 et le paramètre est le taux d'expression ou la stabilité du polynucléotide.

8. Système de criblage de la revendication 7, dans lequel le réactif à base de ABHD6 est le polypeptide ABHD6 et le paramètre est l'activité lipase de ABHD6.

9. Système de criblage de la revendication 7, dans lequel le réactif à base de ABHD6 est le polynucléotide codant pour un polypeptide ABHD6 et le paramètre est le taux d'expression du polynucléotide.

10. Système de criblage de l'une quelconque des revendications 7 à 9, dans lequel la cuve de réaction comprend une cellule comportant le réactif à base de ABHD6.

11. Système de criblage de l'une quelconque des revendications 7 à 10, dans lequel la valeur témoin est au moins l'un de : le paramètre du réactif à base de ABHD6 en l'absence de l'agent, le paramètre du réactif à base de ABHD6 en présence d'un agent témoin qui ne produit pas d'augmentation de sécrétion d'insuline chez le sujet et une valeur prédéterminée associée à une absence d'augmentation de sécrétion d'insuline.

12. Système de criblage de l'une quelconque des revendications 7 à 11, dans lequel le sujet souffre d'au moins l'une des affections suivantes : le diabète, de préférence le diabète de type II, et le syndrome métabolique X.

13. Produit logiciel mis en oeuvre sur un support lisible par ordinateur et comprenant des instructions pour caractériser la capacité d'un agent à augmenter la sécrétion d'insuline chez un sujet, ledit produit comprenant :
(a) un module de réception pour recevoir une valeur d'un paramètre d'un réactif à base de ABHD6 en présence de l'agent dans une cuve de réaction ;
(b) un module de comparaison pour déterminer si la valeur du paramètre du réactif à base de ABHD6 en présence de l'agent est inférieure, égale ou supérieure à une valeur témoin et générer une sortie correspondante ;
(c) un module de caractérisation recevant la sortie correspondante depuis le module de comparaison et adapté pour caractériser l'utilité de l'agent pour augmenter la sécrétion d'insuline, dans lequel :
i. l'agent est caractérisé comme ayant la capacité à augmenter la sécrétion d'insuline chez le sujet lorsque la valeur du paramètre du réactif à base de ABHD6 est inférieure à la valeur témoin ; et
ii. l'agent est caractérisé comme n'ayant pas la capacité à augmenter la sécrétion d'insuline chez le sujet lorsque la valeur du paramètre du réactif à base de ABHD6 est égale ou supérieure à la valeur témoin ;
dans lequel le réactif à base de ABHD6 est un polypeptide ABHD6 et le paramètre est l'activité lipase, la quantité ou la stabilité du polypeptide ABHD6 ; ou
dans lequel le réactif à base de ABHD6 est un polynucléotide codant pour le polypeptide ABHD6 et le paramètre est le taux d'expression ou la stabilité du polynucléotide.

14. Produit logiciel de la revendication 13, dans lequel le réactif à base de ABHD6 est le polypeptide ABHD6 et le paramètre est l'activité lipase de ABHD6.

15. Produit logiciel de la revendication 13, dans lequel le réactif à base de ABHD6 est le polynucléotide codant pour un polypeptide ABHD6 et le paramètre est le taux d'expression du polynucléotide.

16. Produit logiciel de l'une quelconque des revendications 13 à 15, dans lequel la cuve de réaction comprend une cellule contenant le réactif à base de ABHD6.

17. Produit logiciel de l'une quelconque des revendications 13 à 16, dans lequel la valeur témoin est au moins l'un de : le paramètre du réactif à base de ABHD6 en l'absence de l'agent, le paramètre du réactif à base de ABHD6 en présence d'un agent témoin qui ne produit pas d'augmentation de la sécrétion d'insuline chez le sujet et une valeur prédéterminée associée à une absence d'augmentation de sécrétion d'insuline.

18. Produit logiciel de l'une quelconque des revendications 13 à 17, dans lequel le sujet souffre d'au moins l'une des affections suivantes : le diabète, de préférence le diabète de type II, et le syndrome métabolique X.
